# EUROPEAN PATENT SPECIFICATION

(11) **EP 3 563 857 B1**
(45) Date of publication and mention of the grant of the patent: **26.04.2023**
(21) Application number: 17888752.7
(22) Date of filing: 25.12.2017
(51) Int. Cl.: A61K 35/28, A61P 1/16, A61P 13/12, C12N 5/00, A61P 43/00, C12N 5/0775, A61L 27/38

(54) **REPAIR AGENT FOR LIVING TISSUE DAMAGE COMPRISING MESENCHYMAL STEM CELLS, AND METHOD FOR PRODUCING SAID REPAIR AGENT**
REPARATURMITTEL, ENTHALTEND MESENCHYMALE STAMMZELLEN, FÜR SCHÄDEN AN LEBENDEN GEWEBEN UND VERFAHREN ZUR HERSTELLUNG DES REPARATURMITTELS
AGENT DE RÉPARATION POUR LÉSION DE TISSU VIVANT COMPRENANT DES CELLULES SOUCHES MÉSENCHYMATEUSES, ET PROCÉDÉ DE PRODUCTION DUDIT AGENT DE RÉPARATION

(30) Priority: 28.12.2016 JP 2016256779; 27.09.2017 JP 2017187074
(43) Date of publication of application: 06.11.2019
(73) Proprietor: Two Cells Co., Ltd, Hiroshima-shi, Hiroshima 732-0816 (JP); Hiroshima University, Higashi-Hiroshima-shi Hiroshima 739-8511 (JP)
(72) Inventor: KATO, Yukio, Hiroshima-shi Hiroshima 732-0816 (JP); TSUJI, Koichiro, Hiroshima-shi Hiroshima 732-0816 (JP); NAKASHIMA, Ayumu, Hiroshima-shi Hiroshima 734-8553 (JP); MASAKI, Takao, Hiroshima-shi Hiroshima 734-8551 (JP); DOI, Shigehiro, Hiroshima-shi Hiroshima 734-8551 (JP); YOSHIDA, Ken, Hiroshima-shi Hiroshima 734-8553 (JP)
(74) Representative: Plasseraud IP
(86) International application number: PCT/JP2017/046427
(87) International publication number: WO 2018/123968

(56) References cited:
- EP-A1- 3 563 857
- WO-A1-2007/080919
- WO-A1-2011/111787
- WO-A1-2015/016357
- CN-A- 104 666 347
- JP-A- 2011 525 355
- JP-A- 2012 157 263
- JP-A- 2016 525 098
- YOON OK JANG ET AL: "Effect of bone marrow-derived mesenchymal stem cells on hepatic fibrosis in a thioacetamide-induced cirrhotic rat model", BMC GASTROENTEROLOGY, BIOMED CENTRAL LTD., LONDON, GB, vol. 14, no. 1, 25 November 2014 (2014-11-25), page 198, XP021205828, ISSN: 1471-230X, DOI: 10.1186/S12876-014-0198-6
- WANG, S. et al.: "Tumor necrosis factor-inducible gene 6 promotes liver regeneration in mice with acute liver injury", Stem cell research & therapy, vol. 6, 11 March 2015 (2015-03-11), page 20, XP021215848, ISSN: 1757-6512
- MARUYAMA, SHOICHI et al.: "Toward Development of Adipose-derived Stem Cells for the Treatment of Intractable Renal Disease", The Current Medicine, vol. 61, no. 2, 2013, pages 177-184, XP009515551, ISSN: 0433-3047

## Description

### Technical Field

The present disclosure relates to a repair agent for damaged tissue, and to a method for producing the repair agent.

### Background Art

Mesenchymal stem cells (MSCs) can be isolated not only from tissues such as bone marrow, fat, a synovial membrane, an alveolar bone, and a periodontal membrane, but also from various tissues such as a placenta, cord blood, and an umbilical cord. In addition, the mesenchymal stem cells can be cultured and proliferated in vitro. Furthermore, the mesenchymal stem cells have multipotency that allows the mesenchymal stem cells to differentiate not only into mesenchymal cells (e.g., osteoblasts, fat cells, and cartilage cells) but also into non-mesenchymal cells (e.g., neural precursor cells and hepatocytes). Thus, the mesenchymal stem cells are expected to be used as a material for production of cells for use in regenerative medicine and cell therapy.

The mesenchymal stem cells can be cultured by, for example, not only using a medium containing fetal bovine serum (FBS) but also using a serum-free medium in which fewer heterozoic proteins are mixed. For example, Patent Literatures 1 to 3 each describe serum-free culture for use in culture of mesenchymal stem cells.

### Citation List

### [Patent Literatures]

[Patent Literature 1] International Publication No. 2007/080919 (Publication Date: July 19, 2007)
[Patent Literature 2] International Publication No. 2011/111787 (Publication Date: September 15, 2011)
[Patent Literature 3] International Publication No. 2015/016357 (Publication Date: February 5, 2015)

### Summary of Invention

The scope of the invention is defined by the claims. Any subject-matter falling outside the scope of the claims is provided for information purposes only. Any references in the description to methods of treatment in the subsequent paragraphs of this description are to be interpreted as references to refer to the compounds, pharmaceutical compositions and medicaments of the present invention for use in a method for treatment of the human (or animal) body by therapy (or for diagnosis).

### Technical Problem

Mesenchymal stem cells have a wide variety of functions. However, only part of those functions of the mesenchymal stem cells are currently being used. Thus, the mesenchymal stem cells are expected to have many unknown functions.

An aspect of the present disclosure has been made in view of the problems, and an object of an aspect of the present disclosure is to provide (i) a repair agent for damaged tissue of a living body and (ii) a method for producing the repair agent.

### Solution to Problem

The inventors of the present disclosure accomplished the present disclosure by finding, during a process for diligently studying an influence of mesenchymal stem cells on damaged tissue, (1) that mesenchymal stem cells cultured in a serum-free medium containing a specific component have damaged tissue repairing effects (e.g., a fibrosis suppressing effect, an inflammatory cell infiltration suppressing effect, and a macrophage activity controlling effect) which are much more powerful than those of mesenchymal stem cells cultured in a serum medium and (2) that a larger amount of TSG-6 is expressed in mesenchymal stem cells cultured in a serum-free medium than in mesenchymal stem cells cultured in a serum medium.

In order to attain the object, a repair agent for damaged tissue of an aspect of the present disclosure contains mesenchymal stem cells cultured in a serum-free medium.

In order to attain the object, a method of an aspect of the present dislosure for producing a repair agent for damaged tissue includes the step of: (a) culturing mesenchymal stem cells in a serum-free medium.

A repair agent for damaged tissue of an aspect of the present disclosure contains TNF-stimulated gene 6 protein (TSG-6), the damaged tissue being tissue damage that accompanies chronic renal failure, a chronic kidney disease, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, or fibrosis.

Note that mesenchymal stem cells contained in a repair agent for damaged tissue of an aspect of the present disclosure are defined by a production process such as a process of "being cultured in a serum-free medium". The inventors of the present disclosure carried out a test on presence or absence of genes (i.e., gene markers) which differ in expression level between mesenchymal stem cells for use in an aspect of the present disclosure and conventional mesenchymal stem cells (specifically, mesenchymal stem cell cultured in a serum medium). A result of the test made it clear that an enormous variety of genes differ in expression level. However, of these genes, only TSG-6 was finally defined as a gene which is important in distinguishing between mesenchymal stem cells for use in an aspect of the present disclosure and conventional mesenchymal stem cells. An enormous amount of time and effort is further necessary to define what gene is important in distinguishing between mesenchymal stem cells for use in an aspect of the present disclosure and conventional mesenchymal stem cells. Thus, the inventors of the present disclosure concluded that there exist circumstances under which directly defining mesenchymal stem cells of a repair agent for damaged tissue of an aspect of the present disclosure by its structure or properties at the time of filing the present application is impossible or impractical.

### Advantageous Effects of disclosure

Mesenchymal stem cell for use in an aspect of the present disclosure have a tissue fibrosis suppressing effect, an inflammatory cell infiltration suppressing effect, and an inflammation-suppressing macrophage increasing effect which are much more powerful than those of mesenchymal stem cells cultured in a serum medium. Furthermore, mesenchymal stem cells for use in an aspect of the present disclosure have an effect of relieving inflammation early by hyperexpression of TSG-6. Thus, an aspect of the present disclosure brings about a damaged tissue recovering effect which cannot be achieved in a case where mesenchymal stem cells cultured in a serum medium are used and which is remarkably powerful (i.e., so powerful as to be available for a clinical site).

### Brief Description of Drawings

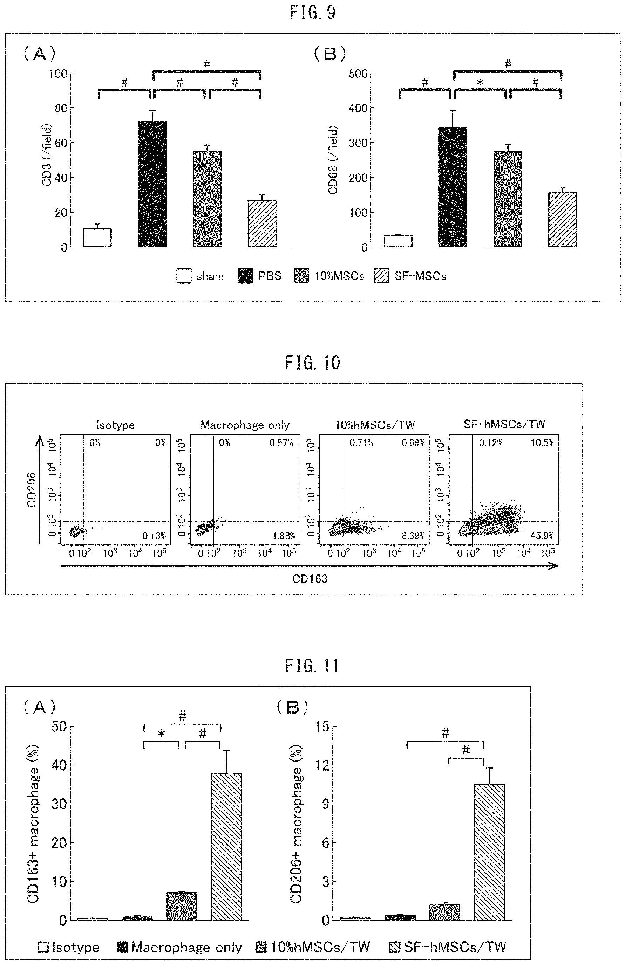

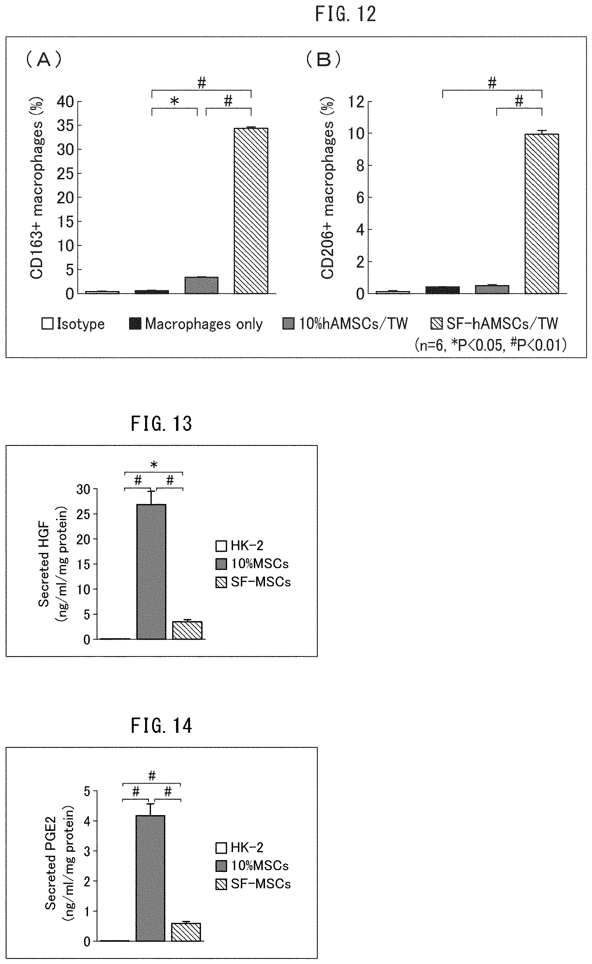

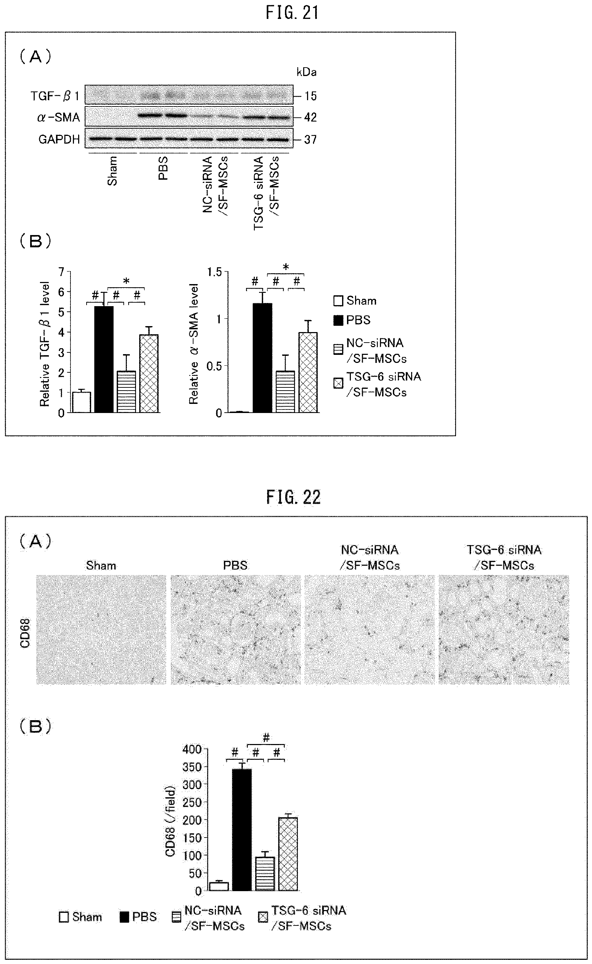

Fig. 1 shows graphs each showing a TGF-β1 mRNA expression level.
(A) of Fig. 2 shows an image showing a result of detection of a TGF-β1 by Western blotting. (B) of Fig. 2 shows graphs each showing a TGF-β1 protein expression level.
Fig. 3 shows an image showing a result of fluorescent immunostaining carried out in an Example of the present disclosure.
Fig. 4 shows graphs each showing an α-SMA mRNA expression level.
(A) of Fig. 5 shows an image showing a result of detection of α-SMA by Western blotting. (B) of Fig. 5 shows graphs each showing an α-SMA protein expression level.
(A) of Fig. 6 shows an image showing a result of immunostaining carried out in an Example of the present disclosure. (B) of Fig. 6 shows graphs each showing a result of a count of the number of α-SMA positive cells per unit area of the image shown in (A) of Fig. 6.
(A) of Fig. 7 shows graphs each showing a CD3 mRNA expression level. (B) of Fig. 7 shows graphs each showing a CD68 mRNA expression level.

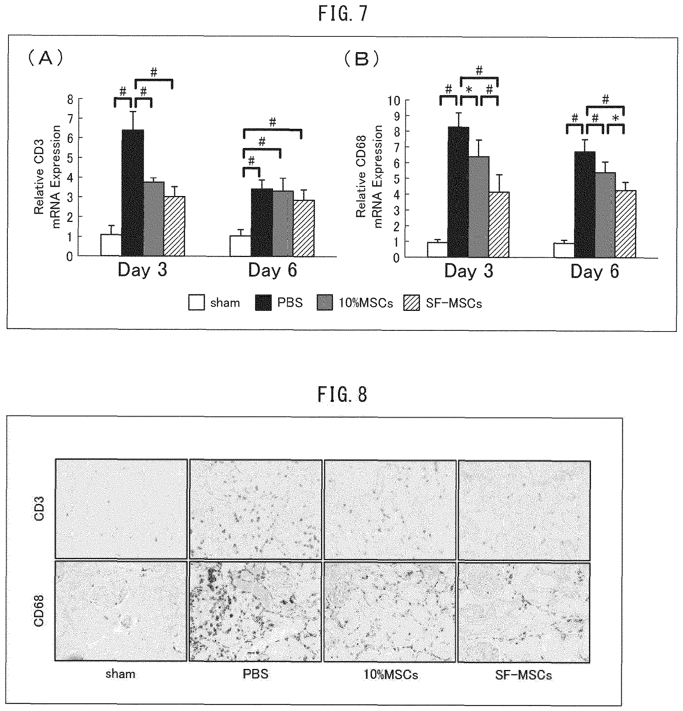

Fig. 8 shows an image showing a result of immunostaining carried out in an Example of the present disclosure.
(A) of Fig. 9 shows graphs each showing a result of a count of the number of CD3 positive cells per unit area of the image shown in Fig. 8. (B) of Fig. 9 shows each graphs showing a result of a count of the number of CD68 positive cells per unit area of the image shown in Fig. 8.
Fig. 10 is a view showing induction of differentiation from an M1-type macrophage into an M2-type macrophage.
(A) of Fig. 11 shows graphs showing an increase in CD 163 positive cell. (B) of Fig. 11 shows graphs showing an increase in CD206 positive cell.
(A) of Fig. 12 shows graphs showing an increase in CD163 positive cell. (B) of Fig. 12 shows graphs showing an increase in CD206 positive cell.
Fig. 13 shows graphs each showing an HGF protein expression level.
Fig. 14 shows graphs each showing a PGE2 protein expression level.

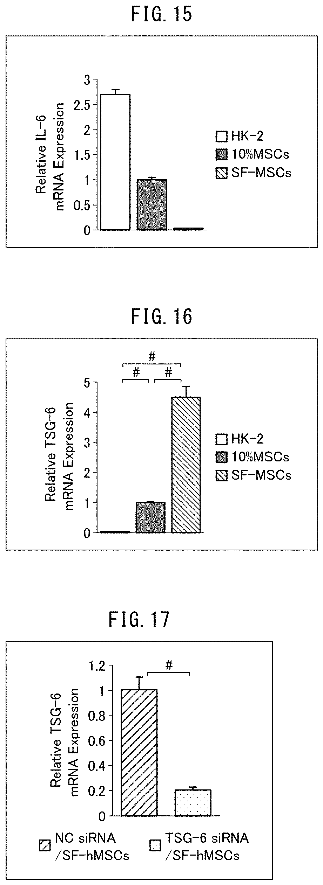

Fig. 15 shows graphs each showing an IL-6 mRNA expression level.
Fig. 16 shows graphs each showing a TSG-6 mRNA expression level.
Fig. 17 shows graphs each showing an expression level of mRNA of TSG-6.

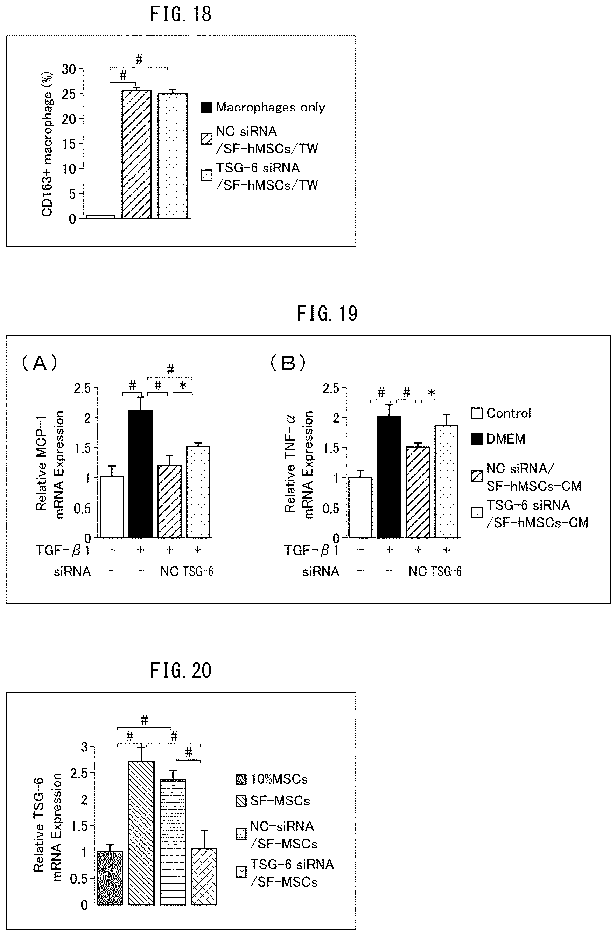

Fig. 18 shows graphs showing an increase in CD163 positive cell.
(A) of Fig. 19 shows graphs each showing an MCP-1 mRNA expression level. (B) of Fig. 19 shows graphs each showing a TNF-α mRNA expression level.
Fig. 20 shows graphs each showing a TSG-6 mRNA expression level.
(A) of Fig. 21 shows an image showing a result of detection of a TGF-β1 and α-SMA by Western blotting. (B) of Fig. 21 shows graphs showing TGF-β expression levels and α-SMA expression levels.
(A) of Fig. 22 shows an image showing a result of immunostaining of CD68. (B) of Fig. 22 shows graphs each showing the number of cells in which CD68 is expressed.

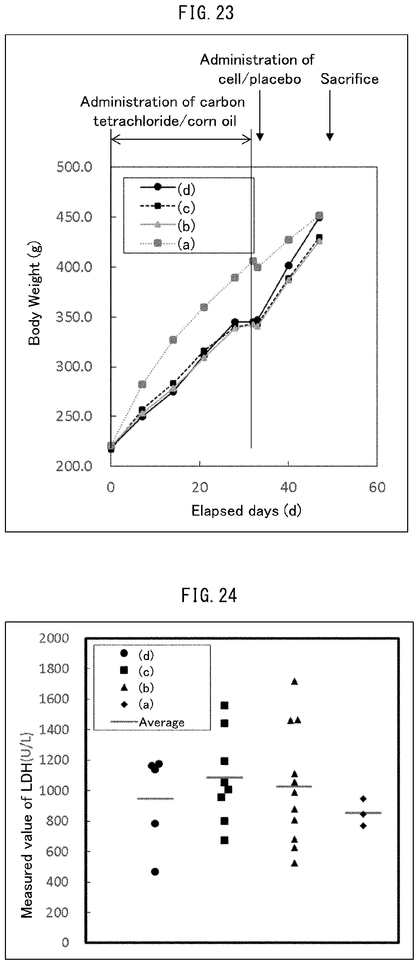

Fig. 23 shows graphs each showing a change in body weight of a model rat in an Example of the present disclosure.
Fig. 24 is a graph showing values of LDH of a model rat in an Example of the present disclosure.

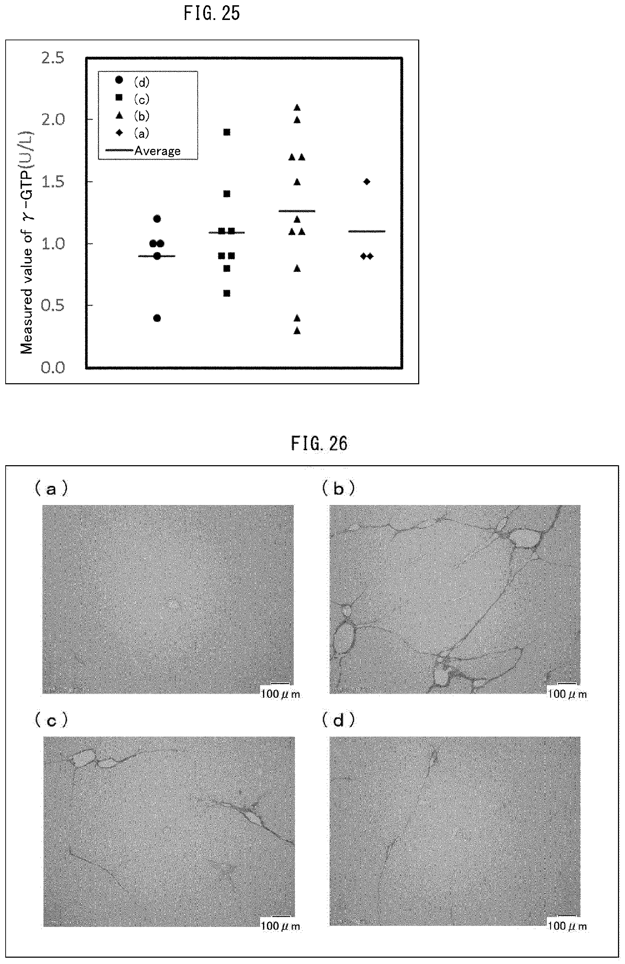

Fig. 25 is a graph showing values of γ-GTP of a model rat in an Example of the present disclosure.
Fig. 26 shows images of a model rat stained with Sirius red in an Example of the present disclosure.

### Description of Embodiments

An embodiment of the present disclosure is specifically described below. Note, however, that the present disclosure is not limited to the above embodiment but can be altered by a skilled person in the art within the scope of the claims. Note that any numerical range expressed as "A to B" herein means "not less than A and not more than B" unless otherwise specified herein.

### [1-1. Method for producing repair agent]

A method in accordance with an embodiment of the present disclosure for producing a repair agent for damaged tissue (hereinafter also referred to as "a production method of an embodiment of the present disclosure") is a method including the step of culturing mesenchymal stem cells in a serum-free medium.

The serum-free medium is not particularly limited in composition provided that the serum-free medium makes it possible to culture mesenchymal stem cells. The serum-free medium can be a publicly known serum-free medium as appropriate. Examples of the publicly known serum-free medium include STK1 (manufactured by TWOCELLS COMPANY, LIMITED), STK2 (manufactured by TWOCELLS COMPANY, LIMITED), a kit for a complete synthetic medium dedicated for human mesenchymal stem cells (MSCGM-CD BulletKit) (Lonza), Mesenchymal Stem Cell Growth Medium DXF (Ready-touse) (PromoCell GmbH.), Stem Pro MSC SFM Xeno free (Thermo Fisher Scientific Inc.), and MesenCult-ACF Medium Kit (STEMCELL Technologies Inc.).

Specific examples of the serum-free medium include (i) a serum-free medium containing an FGF, a PDGF, an EGF, at least one phospholipid, and at least one fatty acid, (ii) a serum-free medium containing an FGF, a PDGF, an EGF, a transforming growth factor-β (TGF-β), at least one phospholipid, and at least one fatty acid, (iii) a serum-free medium containing an FGF, a PDGF, an EGF, dexamethasone, insulin, serum albumin, at least one phospholipid, and at least one fatty acid, and (iv) a serum-free medium containing an FGF, a PDGF, an EGF, a TGF-β, dexamethasone, insulin, serum albumin, at least one phospholipid, and at least one fatty acid.

Note that the serum-free medium (i) and the serum-free medium (iii) can each be a serum-free medium containing no TGF-β and/or no HGF. Note also that the serum-free medium (ii) and the serum-free medium (iv) can each be a serum-free medium containing no HGF.

The phospholipid is exemplified by, but not particularly limited to, phosphatidic acid, lysophosphatidic acid, phosphatidylinositol, phosphatidyl serine, phosphatidyl ethanolamine, phosphatidyl choline, and phosphatidyl glycerol. Those phospholipids can each be used alone, or two or more of those phospholipids can be used in combination (for example, phosphatidic acid and phosphatidyl choline can be used in combination). Those phospholipids can be derived from animals or plants.

The fatty acid is exemplified by, but not particularly limited to, linoleic acid, oleic acid, linolenic acid, arachidonic acid, myristic acid, palmitoyl acid, palmitic acid, and stearic acid. Those fatty acids can each be used alone, or two or more of those fatty acids can be used in combination.

A serum-free medium for use in an embodiment of the present disclosure optionally can contain component(s) different from the above-mentioned components, such as cholesterol and/or a hepatocyte growth factor (HGF). An HGF is contained at a final concentration of preferably 0.1 ng/ml to 50 ng/ml, and more preferably 5 ng/ml, relative to a basal medium (described later). It is a matter of course that such a component(s) is/are not essential to an aspect of the present disclosure.

The production method of an embodiment of the present disclosure is more specifically described below.

### (A. First step)

According to the production method of an embodiment of the present disclosure, mesenchymal stem cells are cultured in a serum-free medium containing an FGF, a PDGF, an EGF, at least one phospholipid, and at least one fatty acid, the serum-free medium more preferably further containing at least any one of dexamethasone, insulin, and serum albumin (the step of thus culturing mesenchymal stem cells is hereinafter referred to as a "first step"). Note that the serum-free medium can be a serum-free medium containing no TGF-β and/or no HGF.

The serum-free medium which contains at least any one of dexamethasone, insulin, and serum albumin brings about an effect of (i) extending a lifetime of mesenchymal stem cells and (ii) accelerating proliferation of mesenchymal stem cells.

A basal medium for constituting the serum-free medium for use in the first step is not limited to any particular basal medium provided that the basal medium is a medium for an animal cell which medium is well known in the present field. Preferable examples of the basal medium include a Ham's F12 medium, a DMEM medium, an RPMI-1640 medium, and an MCDB medium. Those basal media can each be used alone, or a mixture of two or more of those basal media can be used. In an embodiment, a basal medium for constituting the serum-free medium is preferably a medium in which MCDB and DMEM are mixed at a ratio of 1:1. In an embodiment, the serum-free medium which is obtained by adding an FGF, a PDGF, an EGF, at least one phospholipid, and at least one fatty acid to the basal medium can be used in the first step.

An FGF is contained at a final concentration of preferably 0.1 ng/ml to 100 ng/ml, and more preferably 3 ng/ml, relative to the basal medium. A PDGF is contained at a final concentration of preferably 0.5 ng/ml to 100 ng/ml, and more preferably 10 ng/ml, relative to the basal medium. An EGF is contained at a final concentration of preferably 0.5 ng/ml to 200 ng/ml, and more preferably 20 ng/ml, relative to the basal medium. A phospholipid(s) is/are contained in total at a final concentration of preferably 0.1 pg/ml to 30 pg/ml, and more preferably 10 pg/ml, relative to the basal medium. A total amount of a fatty acid(s) contained relative to the basal medium is preferably 1/1000 to 1/10, and more preferably 1/100 of a weight of the basal medium.

Dexamethasone is contained at a final concentration of preferably 10⁻¹⁰ M to 10⁻⁵ M, and more preferably 10⁻⁹ M to 10⁻⁶ M, relative to the basal medium. Insulin is contained at a final concentration of preferably 0.01 pg/ml to 500 pg/ml, and more preferably 0.1 pg/ml to 50 pg/ml, relative to the basal medium. Serum albumin is contained at a final concentration of preferably 0.01 mg/ml to 50 mg/ml, and more preferably 0.1 mg/ml to 5 mg/ml, relative to the basal medium.

By using such a serum-free medium, it is possible to obtain a proliferation promoting effect equal to or greater than that of a serum-containing medium while preventing a heteroprotein from being mixed into the serum-free medium. This allows mesenchymal stem cells to be proliferated as desired.

A phospholipid and a fatty acid each contained in the serum-free medium can be a specific phospholipid and a specific fatty acid, respectively, each of which has already been described.

As used herein, an FGF means a growth factor selected from a fibroblast growth factor (FGF) family, and is preferably an FGF-2 (bFGF). Alternatively, an FGF can be, for example, an FGF-1 selected from another FGF family. As used herein, a PDGF means a growth factor selected from a platelet derived growth factor (PDGF) family, and is preferably a PDGF-BB or a PDGF-AB.

An EGF means a growth factor selected from an epidermal growth factor (EGF) family.

In an embodiment, the serum-free medium can further contain at least two factors selected from the group consisting of a connective tissue growth factor (CTGF), a vascular endothelial growth factor (VEGF), and an ascorbic acid compound.

As used herein, an ascorbic acid compound means ascorbic acid (vitamin C), ascorbate diphosphate, or a compound similar to ascorbic acid or ascorbate diphosphate.

In an aspect, the serum-free medium preferably contains a lipid antioxidant. In an embodiment, the lipid antioxidant contained in the serum-free medium can be DL-α-tocopherol acetate (vitamin E). The serum-free medium can further contain a surfactant. In an embodiment, the surfactant contained in the serum-free medium can be Pluronic F-68 or Tween 80.

The serum-free medium can further contain insulin, transferrin, dexamethasone, serum albumin, and selenate. As used herein, insulin can be an insulin-like growth factor, can be derived from a natural cell, or can be produced by genetic modification.

In the first step, mesenchymal stem cells isolated from animal tissues such as human tissues by a conventionally publicly-known method are seeded into the serum-free medium (described earlier) and are cultured until the number of mesenchymal stem cells proliferated reaches the desired number of mesenchymal stem cells (it is a matter of course that in the first step, the mesenchymal stem cells can alternatively be merely maintained in the serum-free medium without being proliferated). It is preferable that mesenchymal stem cells separated from 1 mg to 500 mg of a tissue piece(s) (containing mesenchymal stem cells) be seeded per 1 ml of a medium at a culture temperature of 37°C±1°C and under 5% CO₂. Note that a culture time is not limited to any particular culture time provided that the culture time allows a concentration of the mesenchymal stem cells to reach an intended concentration. For example, the culture time can be 1 hour to 5 hours, 1 hour to 10 hours, 1 hour to 20 hours, 1 hour to 1 day, 1 hour to 10 days, 1 hour to 30 days, 1 hour to 50 days, 1 hour to 60 hours, 1 hour to 70 hours, 30 hours to 70 hours, 40 hours to 70 hours, or 50 hours to 70 hours.

The mesenchymal stem cells which are to be subjected to the first step are preferably exemplified by, but not particularly limited to, initial mesenchymal stem cells, i.e., cells which have never been subcultured since the cells were collected from animal tissues such as human tissues.

Furthermore, in order to more efficiently proliferate the mesenchymal stem cells in the first step, it is preferable to use a culture vessel suitable for culture to carry out the first step with respect to each of mesenchymal stem cells to be cultured in the first step (hereinafter also referred to as "first step target cells"). Examples of a method for selecting a culture vessel suitable for culture of first step target cells include a method in which an optimum culture vessel is selected by first step target cells. Specifically, a plurality of types of culture vessels are prepared, and first step target cells are proliferated under the same condition except that the culture vessels differ in type. After two weeks have elapsed from the start of culture, the number of cells held by each of the culture vessels is measured by a publicly-known method. Then, it can be determined that a culture vessel holding the most cells is the most suitable for culture of the first step target cells. Further, in a case where the first step target cells are proliferated at a high speed, it can be determined, even before two weeks have elapsed from the start of culture, that a culture vessel in which the number of first step target cells reaches 80% to 90% of the number of cells in a confluent state in the shortest period is the most suitable for culture of the first step target cells.

In the first step of the production method of an embodiment of the present disclosure, in a case where a culture vessel suitable for proliferation of the first step target cells has been made clear, it is possible to use that culture vessel. In contrast, in a case where a culture vessel suitable for culture of the first step target cells has not been made clear, the production method of an embodiment of the present disclosure can further include, before the first step, a "culture vessel selecting step" for selecting a culture vessel suitable for culture of the first step target cells.

Note that adhesion of the mesenchymal stem cells to a culture vessel is a requisite to proliferation of the mesenchymal stem cells. Thus, in a case where the mesenchymal stem cells less strongly adhere to the culture vessel, the serum-free medium is preferably caused, in the first step, to further contain cell adhesion molecules. Examples of the "cell adhesion molecules" include fibronectin, collagen, and gelatin. Those cell adhesion molecules can be used alone in one kind or can be used in combination of two or more kinds.

In the first step, the mesenchymal stem cells can be subcultured at least once. The mesenchymal stem cells are anchorage-dependently proliferated. Thus, for example, in a case where the mesenchymal stem cells are locally unevenly proliferated, by subculturing the mesenchymal stem cells in the process of carrying out the first step, it is possible to culture the mesenchymal stem cells under a better condition. Note that it is preferable that the culture first step is preferably carried out during a period from primary culture (P0) to third passage culture (P3).

The mesenchymal stem cells do not necessarily need to be subcultured by any particular method, but can be subcultured by a conventionally publicly-known method for subculturing mesenchymal stem cells. In order that mesenchymal stem cells which have been subcultured will be in good condition, the mesenchymal stem cells which are being subcultured in the first step are preferably detached with use of a cell detaching agent containing neither a mammal-derived component nor a microorganism-derived component. Examples of the "cell detaching agent containing neither a mammal-derived component nor a microorganism-derived component" include ACCUTASE (Innovative Cell Technologies, Inc.).

The following description discusses an example of a subculture method which is carried out by using ACCUTASE as the "cell detaching agent containing neither a mammal-derived component nor a microorganism-derived component". The mesenchymal stem cells are detached and subcultured by a process including the steps (i) to (vi) below. Note that the following describes a subculture method which is carried out by using a T-25 flask (manufactured by Falcon) as a culture vessel.
(i) A cell layer on the flask is washed with 5 mL of PBS (-).
(ii) To the cell layer, 2 mL of ACCUTASE is added.
(iii) The cell layer is caused to stand still at a room temperature for approximately two minutes, and after it is confirmed that cells have been detached from the flask, the PBS (-) which contains the cells thus detached is transferred to a centrifuging tube.
(iv) 7 mL of PBS (-) is added to the flask so that a bottom surface of the flask is rinsed with that PBS (-).
(v) A resultant solution obtained in the step (iv) is transferred to the centrifuging tube used in the step (iii), and the solution is centrifuged at 1500 rpm (200 ×g to 1000 ×g) for five minutes.
(vi) Supernatant is removed from the centrifuging tube, and cells are seeded into a serum-free medium in a new flask at a seeding density of 5,000 cells/cm².

### (B. Second step)

The production method of an embodiment of the present disclosure can further include, after the first step, a second step of culturing mesenchymal stem cells in a serum-free medium containing an FGF, a PDGF, a TGF-β, an EGF, at least one phospholipid, and at least one fatty acid, the serum-free medium more preferably further containing at least any one of dexamethasone, insulin, and serum albumin. Note that the serum-free medium can be a serum-free medium containing no HGF.

Note here that the "serum-free medium" used in the second step differs from the serum-free medium (described earlier in (A. First step)) in that the "serum-free medium" used in the second step contains a TGF-β. The serum-free medium used in the first step and the serum-free medium used in the second step may be herein referred to as a "serum-free medium A" and a "serum-free medium B", respectively. Since components except a TGF-β (e.g., an FGF, a PDGF, an EGF, dexamethasone, insulin, serum albumin, at least one phospholipid, and at least one fatty acid) and a basal medium are as described earlier in (A. First step) in which the serum-free medium A is discussed, a description thereof is omitted here. Furthermore, respective amounts of the above components contained in the serum-free medium B used in the second step can be identical to the respective amounts (described earlier) of the above components contained in the serum-free medium A.

In an embodiment, a TGF-β is contained at a final concentration of preferably 0.5 ng/ml to 100 ng/ml, and more preferably 10 ng/ml, relative to the basal medium.

In the second step, mesenchymal stem cells obtained through the first step are seeded into the serum-free medium B (described earlier) and are cultured until the number of mesenchymal stem cells proliferated reaches the desired number of mesenchymal stem cells (it is a matter of course that in the second step, the mesenchymal stem cells can alternatively be merely maintained in the serum-free medium B without being proliferated). It is preferable that 1×10⁴ to 2×10⁴ mesenchymal stem cells be seeded per 1 ml of a medium at a culture temperature of 37°C±1°C and under 5% CO₂. Note that a cell culture time is not limited to any particular cell culture time provided that the cell culture time is long enough to obtain an intended cell concentration. For example, the culture time can be 1 hour to 5 hours, 1 hour to 10 hours, 1 hour to 20 hours, 1 hour to 1 day, 1 hour to 10 days, 1 hour to 30 days, 1 hour to 50 days, 1 hour to 60 hours, 1 hour to 70 hours, 30 hours to 70 hours, 40 hours to 70 hours, or 50 hours to 70 hours. By thus culturing the mesenchymal stem cells, it is possible to efficiently obtain a large number of mesenchymal stem cells whose ability to recover from tissue damage is maintained or improved.

In the second step, a culture vessel for use in culture is not limited to any particular culture vessel provided that the culture vessel allows the mesenchymal stem cells to be proliferated. Preferable examples of the culture vessel include a 75 cm² flask (manufactured by Falcon) and a 75 cm² flask (manufactured by SUMITOMO BAKELITE CO., LTD.). Note, however, that culture of some cells may be affected by a type of a culture vessel used. Thus, in order to more efficiently culture the mesenchymal stem cells, it is preferable to use a culture vessel suitable for culture to carry out the second step with respect to each of mesenchymal stem cells to be cultured (hereinafter also referred to as "culture target cells"). Since a method for selecting a culture vessel suitable for proliferation of second step target cells is as described earlier in (A. First step), a description thereof is omitted here.

In a case where the mesenchymal stem cells less strongly adhere to the culture vessel, the serum-free medium B can further contain cell adhesion molecules. Since the cell adhesion molecules are as described earlier in (A. First step), a description thereof is omitted here.

In the second step, the mesenchymal stem cells can be subcultured at least once. By subculturing the mesenchymal stem cells in the process of carrying out the second step, it is possible to culture the mesenchymal stem cells under a better condition.

Note that mesenchymal stem cells which have been subcultured at least once (P1) since the mesenchymal stem cells were collected from animal tissues such as human tissues are preferably subjected to the second step.

Since (i) a method for subculturing the mesenchymal stem cells in the process of carrying out the first step and (ii) a subculture method carried out in a case where cells which have been obtained through the first step are subjected to the second step are as described earlier in (A. First step), a description thereof is omitted here.

The production method of an embodiment of the present disclosure can further include, before the second step (or before the first step), a culture vessel selecting step of selecting a culture vessel suitable for proliferation of mesenchymal stem cells. Since a method for selecting a culture vessel suitable for proliferation of mesenchymal stem cells is as described earlier in (A. First step), a description thereof is omitted here.

As described earlier, the production method of an embodiment of the present disclosure can include the first step and the second step. Note, however, that an aspect of the present disclosure is not limited to such a production method. For example, the production method of an embodiment of the present disclosure can alternatively be a method including only the first step of the first step and the second step or a method including only the second step of the first step and the second step.

### (C. Screening step)

The production method of an embodiment of the present disclosure can further include, after the first step and/or the second step, a screening step of: selecting, from the mesenchymal stem cells by screening, mesenchymal stem cells which are non-tumorigenic.

By using, as a repair agent, the mesenchymal stem cells which have been selected in the screening step, it is possible to achieve a repair agent containing mesenchymal stem cells which are non-tumorigenic, and consequently to achieve a safer repair agent.

In the screening step, it can be investigated by a tumorigenicity test method with use of an in vivo sensitive immunodeficient mouse (NOG mouse) whether the mesenchymal stem cells are tumorigenic.

Specifically, mesenchymal stem cells (e.g., 1,000,000 cells) cultured in the serum-free medium (described earlier) and Hela cells (e.g., 1,000 cells) are subcutaneously-implanted in 10 places in an NOG mouse. In a case where it is impossible to confirm, under a condition (e.g., a specific mouse breeding time) in which it can be confirmed that the Hela cells are tumorigenic (specifically, a tumor has developed in a place in which the Hela cells have been implanted), that the mesenchymal stem cells cultured in the serum-free medium are tumorigenic, it is possible to determine that those mesenchymal stem cells are non-tumorigenic.

### [1-2. Repair agent for damaged tissue]

A repair agent for damaged tissue in accordance with an embodiment of the present disclosure (hereinafter referred to as a "repair agent of an embodiment of the present disclosure") contains mesenchymal stem cells cultured in a serum-free medium.

Note here that the repair agent of an embodiment of the present disclosure means a pharmaceutical agent which has an effect of promoting recovery from tissue damage. The repair agent of an embodiment of the present disclosure can also be referred to as (i) a pharmaceutical agent for suppressing fibrosis of a living tissue (i.e., a fibrosis suppressing agent), (ii) a pharmaceutical agent for suppressing infiltration of an inflammatory cell (i.e., an inflammatory cell infiltration suppressing agent), or (iii) a pharmaceutical agent for controlling activity of a macrophage (i.e., a macrophage activity controlling agent).

The expression "suppressing fibrosis of a living tissue" herein means suppressing tissue hardening caused by (i) abnormal proliferation of a connective tissue or (ii) abnormal accumulation of collagen in a connective tissue. For example, in a case where a living tissue suffers damage, a connective tissue constituting the living tissue may be abnormally proliferated in the process of recovery from the damage. The expression "suppressing fibrosis of a living tissue" means, for example, suppressing, after a living tissue has suffered damage, tissue hardening caused by (i) abnormal proliferation of a connective tissue constituting the living tissue or (ii) abnormal accumulation of collagen in a connective tissue.

The expression "suppressing infiltration of an inflammatory cell" herein means suppressing (i) destruction of a tissue by inflammatory cells (e.g., a macrophage, a lymphocyte, and a neutrophil) or (ii) proliferation of inflammatory cells (e.g., a macrophage, a lymphocyte, and a neutrophil) which enter a tissue.

The expression "controlling macrophage activity" herein means changing a phenotype of a macrophage. The expression "controlling macrophage activity" means, for example, changing a macrophage from a Pro-inflammatory phenotype (M1-type) macrophage which accelerates inflammation of a tissue into an Immune-regulatory phenotype (M2-type) macrophage. By changing a macrophage from an M1-type macrophage into an M2-type macrophage, it is possible to relieve inflammation of a tissue.

Furthermore, the repair agent contains mesenchymal stem cells cultured in a serum-free medium. Note that the term "serum-free medium" herein means a medium which contains no serum, i.e., a medium prepared without addition of any serum. Note also that the term "serum-free culture" means culture with use of a serum-free medium. Mesenchymal stem cells can be cultured by (i) culturing mesenchymal stem cells only in a single serum-free medium or (ii) culturing mesenchymal stem cells in a plurality of serum-free media. Mesenchymal stem cells can be cultured in a plurality of serum-free media by, for example, culturing mesenchymal stem cells in a desired serum-free medium, then replacing the desired serum-free medium with another serum-free medium, and further culturing mesenchymal stem cells in the another serum-free medium.

The serum-free medium is not particularly limited in composition provided that the serum-free medium makes it possible to culture mesenchymal stem cells. The serum-free medium can be a publicly known serum-free medium as appropriate. Examples of the publicly known serum-free medium include STK1 (manufactured by TWOCELLS COMPANY, LIMITED), STK2 (manufactured by TWOCELLS COMPANY, LIMITED), a kit for a complete synthetic medium dedicated for human mesenchymal stem cells (MSCGM-CD BulletKit) (Lonza), Mesenchymal Stem Cell Growth Medium DXF (Ready-touse) (PromoCell GmbH.), Stem Pro MSC SFM Xeno free (Thermo Fisher Scientific Inc.), and MesenCult-ACF Medium Kit (STEMCELL Technologies Inc.).

Specific examples of the serum-free medium include (i) a serum-free medium containing an FGF, a PDGF, an EGF, at least one phospholipid, and at least one fatty acid, (ii) a serum-free medium containing an FGF, a PDGF, an EGF, a TGF-β, at least one phospholipid, and at least one fatty acid, at least one phospholipid, and at least one fatty acid, (iii) a serum-free medium containing an FGF, a PDGF, an EGF, dexamethasone, insulin, serum albumin, at least one phospholipid, and at least one fatty acid, and (iv) a serum-free medium containing an FGF, a PDGF, an EGF, a TGF-β, dexamethasone, insulin, serum albumin, at least one phospholipid, and at least one fatty acid.

The phospholipid is exemplified by, but not particularly limited to, phosphatidic acid, lysophosphatidic acid, phosphatidylinositol, phosphatidyl serine, phosphatidyl ethanolamine, phosphatidyl choline, and phosphatidyl glycerol. Those phospholipids can each be used alone, or two or more of those phospholipids can be used in combination (for example, phosphatidic acid and phosphatidyl choline can be used in combination). Those phospholipids can be derived from animals or plants.

The fatty acid is exemplified by, but not particularly limited to, linoleic acid, oleic acid, linolenic acid, arachidonic acid, myristic acid, palmitoyl acid, palmitic acid, and stearic acid. Those fatty acids can each be used alone, or two or more of those fatty acids can be used in combination.

A serum-free medium for use in an embodiment of the present disclosure optionally can contain component(s) different from the above-mentioned components, such as cholesterol and/or an HGF. It is a matter of course that such a component(s) is/are not essential to an aspect of the present disclosure.

Since respective concentrations of the components contained in the serum-free medium for use in an embodiment of the present disclosure are as described earlier in [1-1. Method for producing repair agent], a description thereof is omitted here.

Mesenchymal stem cells for use in the repair agent of an embodiment of the present disclosure include not only mesenchymal stem cells isolated from tissues such as bone marrow, fat, a synovial membrane, an alveolar bone, and a periodontal membrane, but also mesenchymal stem cells isolated from tissues such as a placenta, cord blood, and an umbilical cord. The mesenchymal stem cells for use in the repair agent of an embodiment of the present disclosure can alternatively be human mesenchymal stem cells (e.g., collected human mesenchymal stem cells) or mesenchymal stem cells derived from non-human animals such as a rat and a mouse.

A living tissue to be treated with the repair agent is exemplified by, but not particularly limited to, a kidney, a liver, a lung, a skin, a cartilage, a bone, the spinal cord, a tooth, a joint, blood vessels (an artery and a vein), a heart, a brain, a jaw, a mouth, an eye, a cornea, and a pharynx. That is, the repair agent can be intended to repair damage to such a living tissue.

A disease on which the repair agent works and which is accompanied by tissue damage is not limited to any particular disease. Examples of the disease include chronic renal failure, a chronic kidney disease, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, and fibrosis.

The repair agent of an embodiment of the present disclosure can be a pharmaceutical agent containing mesenchymal stem cells cultured in a serum-free medium. The repair agent of an embodiment of the present disclosure can contain not only mesenchymal stem cells cultured in a serum-free medium but also a pharmaceutically acceptable additive(s) (e.g., a buffer, an antioxidant, a thickener, and/or an excipient).

An amount of the additive(s) contained is not limited to any particular amount, but can be, for example, 0.01% by weight to 50% by weight, 0.01% by weight to 40% by weight, 0.01% by weight to 30% by weight, 0.01% by weight to 20% by weight, 0.01% by weight to 10% by weight, or 0.01% by weight to 1% by weight, of the repair agent of an embodiment of the present disclosure.

The number of mesenchymal stem cells contained in the repair agent of an embodiment of the present disclosure is not limited to any particular number and can be set as appropriate in accordance with a body weight of a target to which to administer the repair agent. For example, 1×10² mesenchymal stem cells to 1×10¹⁰ mesenchymal stem cells, 1×10³ mesenchymal stem cells to 1×10¹⁰ mesenchymal stem cells, 1×10⁴ mesenchymal stem cells to 1×10¹⁰ mesenchymal stem cells, 1×10⁵ mesenchymal stem cells to 1×10¹⁰ mesenchymal stem cells, or 1×10⁶ mesenchymal stem cells to 1×10¹⁰ mesenchymal stem cells can be contained per dose (per administration). It is a matter of course that 10¹⁰ or more mesenchymal stem cells can be contained per dose (administration).

Examples of a method of administration of the repair agent include an intravenous injection, an intraarterial injection, and local injections (e.g., an injection into the spinal cord (a spinally intracavitary injection), an intramuscular injection, a joint injection, and an injection into the brain). Of these methods of administration of the repair agent, a method of administration of the repair agent, with use of a catheter, to an artery at or near an affected part brings about an advantageous effect of allowing a smaller number of cells to be necessary for administration of the repair agent.

A target to which to administer the repair agent for damaged tissue in accordance with an embodiment of the present disclosure is not limited to any particular target. Examples of the target to which to administer the repair agent include a human and mammals different from the human (e.g., cattle, a pig, a sheep, a goat, a horse, a dog, a cat, a rabbit, a mouse, and a rat).

An amount of administration of the repair agent of an embodiment of the present disclosure is not limited to any particular amount, but can be an amount that causes 1×10² mesenchymal stem cells to 1×10¹⁰ mesenchymal stem cells, 1×10³ mesenchymal stem cells to 1×10¹⁰ mesenchymal stem cells, 1×10⁴ mesenchymal stem cells to 1×10¹⁰ mesenchymal stem cells, 1×10⁵ mesenchymal stem cells to 1×10¹⁰ mesenchymal stem cells, or 1×10⁶ mesenchymal stem cells to 1×10¹⁰ mesenchymal stem cells to be administered per kilogram of body weight of the target to which to administer the repair agent.

### [1-3. Repair agent for damaged tissue-2]

The repair agent of an embodiment of the present disclosure contains TNF-stimulated gene 6 protein (TSG-6), which is an active component.

The inventors of the present disclosure found that a larger amount of TSG-6 is expressed in mesenchymal stem cells cultured in a serum-free medium than in mesenchymal stem cells cultured in a serum medium. Furthermore, the inventors of the present disclosure newly found that TSG-6 has an anti-inflammatory action in various diseases (in particular, a disease in a kidney). The repair agent of an embodiment of the present disclosure makes it possible to, for example, (i) suppress infiltration of an inflammatory cell in an early phase, (ii) relieve inflammation in an early phase, and/or (iii) suppress fibrosis of a living tissue.

The repair agent of an embodiment of the present disclosure preferably contains TSG-6 derived from mesenchymal stem cells cultured in a serum-free medium (more specifically, mesenchymal stem cells themselves cultured in a serum-free medium). Since a repair agent containing TSG-6 derived from mesenchymal stem cells is as specifically described earlier in [1-2. Repair agent for damaged tissue], a description thereof is omitted here.

The repair agent of an embodiment of the present disclosure means a pharmaceutical agent which has an effect of promoting recovery from tissue damage. The repair agent of an embodiment of the present disclosure can be (i) a pharmaceutical agent for suppressing fibrosis of a living tissue (i.e., a fibrosis suppressing agent) or (ii) a pharmaceutical agent for suppressing infiltration of an inflammatory cell (i.e., an inflammatory cell infiltration suppressing agent).

Note here that, since the expressions "suppressing fibrosis of a living tissue" and "suppressing infiltration of an inflammatory cell" are as described earlier in [1-2. Repair agent for damaged tissue], a description thereof is omitted here.

The repair agent of an embodiment of the present disclosure can contain mesenchymal stem cells cultured in a serum-free medium. Since a composition and a concentration of a serum-free medium, a type of mesenchymal stem cell, and/or the like are as described earlier in [1-2. Repair agent for damaged tissue], a description thereof is omitted here.

A living tissue to be treated with the repair agent is exemplified by, but not particularly limited to, a kidney, a liver, a lung, a skin, a cartilage, a bone, the spinal cord, a tooth, a joint, blood vessels (an artery and a vein), a heart, a brain, a jaw, a mouth, an eye, a cornea, and a pharynx. That is, the repair agent can be intended to repair damage to such a living tissue.

A disease on which the repair agent works and which is accompanied by tissue damage is not limited to any particular disease. Examples of the disease include chronic renal failure, a chronic kidney disease, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, and fibrosis.

The repair agent of an embodiment of the present disclosure can be a pharmaceutical agent containing TSG-6 (or mesenchymal stem cells in which TSG-6 is highly expressed and which have been cultured in a serum-free medium). The repair agent of an embodiment of the present disclosure can contain not only TSG-6 (or mesenchymal stem cells in which TSG-6 is highly expressed and which have been cultured in a serum-free medium) but also a pharmaceutically acceptable additive(s) (e.g., a buffer, an antioxidant, a thickener, and/or an excipient).

An amount of additive(s) contained in the repair agent of an embodiment of the present disclosure is exemplified by, but not particularly limited to, 0% by weight to 99.999% by weight, 0% by weight to 99.99% by weight, 0% by weight to 99.9% by weight, 5% by weight to 99.9% by weight, 10% by weight to 99.9% by weight, 20% by weight to 99.9% by weight, 30% by weight to 99.9% by weight, 40% by weight to 99.9% by weight, 50% by weight to 99.9% by weight, 60% by weight to 99.9% by weight, 70% by weight to 99.9% by weight, 80% by weight to 99.9% by weight, and 90% by weight to 99.9% by weight, with respect to 100% by weight of the repair agent.

Examples of a method of administration of the repair agent include an intravenous injection, an intraarterial injection, and local injections (e.g., an injection into the spinal cord (a spinally intracavitary injection), an intramuscular injection, a joint injection, and an injection into the brain). Of these methods of administration of the repair agent, a method of administration of the repair agent, with use of a catheter, to an artery at or near an affected part brings about an advantageous effect of allowing a smaller amount of repair agent to be necessary for administration of the repair agent.

A target to which to administer the repair agent for damaged tissue in accordance with an embodiment of the present disclosure is not limited to any particular target. Examples of the target to which to administer the repair agent include a human and mammals different from the human (e.g., cattle, a pig, a sheep, a goat, a horse, a dog, a cat, a rabbit, a mouse, and a rat).

TSG-6 can be a natural protein, a synthesized protein, or a recombinant protein.

An amount of TSG-6 contained in the repair agent of an embodiment of the present disclosure is exemplified by, but not particularly limited to, 0.001% by weight to 100% by weight, 0.01% by weight to 100% by weight, 0.1% by weight to 100% by weight, 0.1% by weight to 95% by weight, 0.1% by weight to 90% by weight, 0.1% by weight to 80% by weight, 0.1% by weight to 70% by weight, 0.1% by weight to 60% by weight, 0.1% by weight to 50% by weight, 0.1% by weight to 40% by weight, 0.1% by weight to 30% by weight, 0.1% by weight to 20% by weight, and 0.1% by weight to 10% by weight, with respect to 100% by weight of the repair agent.

An aspect of the present disclosure can also be arranged as below.

A repair agent for damaged tissue of an aspect of the present disclosure contains mesenchymal stem cells cultured in a serum-free medium.

The repair agent of an aspect of the present disclosure is preferably arranged such that the repair agent is intended to suppress fibrosis of a living tissue.

The repair agent of an aspect of the present disclosure is preferably arranged such that the repair agent is intended to suppress infiltration of an inflammatory cell.

The repair agent of an aspect of the present disclosure is preferably arranged such that the repair agent is intended to control activity of a macrophage.

The repair agent of an aspect of the present disclosure is preferably arranged such that the serum-free medium contains a fibroblast growth factor (FGF), a platelet-derived growth factor (PDGF), an epidermal growth factor (EGF), at least one phospholipid, and at least one fatty acid.

The repair agent of an aspect of the present disclosure is preferably arranged such that the damaged tissue is tissue damage that accompanies chronic renal failure, a chronic kidney disease, cirrhosis, pulmonary fibrosis, a burn, interstitial pneumonia, drug-induced pneumonia, irradiation pneumonitis, chronic obstructive pulmonary disease, an acute respiratory distress syndrome (ARDS), cartilage damage, a bone defect, spinal cord damage, periodontal disease, multiple sclerosis (MS), rheumatoid arthritis, systemic lupus erythematosus (SLE), Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, or fibrosis.

The repair agent of an aspect of the present disclosure is preferably arranged such that the mesenchymal stem cells are mesenchymal stem cells which have been selected by screening and are non-tumorigenic.

The repair agent of an aspect of the present disclosure is preferably arranged such that the mesenchymal stem cells have been subcultured at least once.

The repair agent of an aspect of the present disclosure is preferably arranged such that the mesenchymal stem cells which are being subcultured are detached with use of a cell detaching agent containing neither a mammal-derived component nor a microorganism-derived component.

The repair agent of an aspect of the present disclosure is preferably arranged such that the mesenchymal stem cells are cultured with use of a culture vessel suitable for culture of the mesenchymal stem cells.

A method of an aspect of the present disclosure for producing a repair agent for damaged tissue includes the step of: (a) culturing mesenchymal stem cells in a serum-free medium.

The method of an aspect of the present disclosure is preferably arranged such that the serum-free medium contains an FGF, a PDGF, an EGF, at least one phospholipid, and at least one fatty acid.

The method of an aspect of the present disclosure is preferably arranged to further include, after the step (a), the step of: (b) selecting, by screening, mesenchymal stem cells which are non-tumorigenic.

The method of an aspect of the present disclosure is preferably arranged such that the mesenchymal stem cells are subcultured at least once in the step (a).

The method of an aspect of the present disclosure is preferably arranged such that the mesenchymal stem cells which are being subcultured are detached with use of a cell detaching agent containing neither a mammal-derived component nor a microorganism-derived component.

The method of an aspect of the present disclosure is preferably arranged such that the mesenchymal stem cells are cultured with use of a culture vessel suitable for culture of the mesenchymal stem cells in the step (a).

A repair agent for damaged tissue of an aspect of the present disclosure contains TNF-stimulated gene 6 protein (TSG-6), the damaged tissue being tissue damage that accompanies chronic renal failure, a chronic kidney disease, cirrhosis, pulmonary fibrosis, a burn, interstitial pneumonia, drug-induced pneumonia, irradiation pneumonitis, chronic obstructive pulmonary disease, an acute respiratory distress syndrome, cartilage damage, a bone defect, spinal cord damage, periodontal disease, multiple sclerosis, rheumatoid arthritis, systemic lupus erythematosus, Crohn's syndrome, diabetes, arteriosclerosis, myocardial infarction, stroke, Alzheimer's disease, macular degeneration, viral hepatitis, alcoholic hepatitis, non-alcoholic steatohepatitis, jawbone reconstruction, cleft palate, a bone replacement material, a bone defect, a bone system disease, a dry eye, a corneal disorder, pharyngitis, arthritis, a cancer, or fibrosis.

The repair agent of an aspect of the present disclosure is preferably arranged such that the repair agent is intended to suppress fibrosis of a living tissue.

The repair agent of an aspect of the present disclosure is preferably arranged such that the repair agent is intended to suppress infiltration of an inflammatory cell.

### Examples

### (Example 1)

Example 1 carried out a study on an effect, brought about by implantation of mesenchymal stem cells cultured in a serum-free medium, of suppressing fibrosis of a kidney.

Mesenchymal stem cells were separated from a femur or the tibia of a Sprague Dawley rat (6 weeks old to 7 weeks old) in accordance with a well-known method (e.g., see Ueno et al., "Mesenchmal stem cells ameliorate experimental peritoneal fibrosis by suppressing inflammation and inhibiting TGF-β signaling" International Society of Nephrology, 2013, p1-11).

The mesenchymal stem cells thus separated were subjected to primary culture for 7 days in a serum-free medium STK1 (manufactured by TWOCELLS COMPANY, LIMITED, corresponding to a "serum-free medium containing an FGF, a PDGF, an EGF, at least one phospholipid, and at least one fatty acid") or a 10% bovine serum-containing DMEM medium (manufactured by Sigma-Aldorich) at 37°C and under 5% CO₂.

Thereafter, the mesenchymal stem cells which had been subjected to primary culture in the serum-free medium STK1 were cultured for 12 days in a serum-free medium STK2 (manufactured by TWOCELLS COMPANY, LIMITED, corresponding to a "serum-free medium containing an FGF, a PDGF, an EGF, a TGF-β, at least one phospholipid, and at least one fatty acid") at 37°C and under 5% CO₂, and the mesenchymal stem cells which had been subjected to primary culture in the 10% bovine serum-containing DMEM medium were cultured for 12 days in a 10% bovine serum-containing DMEM medium at 37°C and under 5% CO₂. Note that ACCUTASE was used for subculture.

Next, as a model for fibrosis of a kidney, a common unilateral ureteral ligation model of a rat was prepared. The unilateral ureteral ligation model was prepared by publicly-known means (e.g., Masaki et al., "Activation of the ERK pathway precedes tubular proliferation in the obstructed rat kidney" Kidney International, Vol.63 (2003), p1256-1264).

Four days after ligation of the ureter, 5×10⁶ mesenchymal stem cells cultured with the STK2 or 5×10⁶ mesenchymal stem cells cultured with the 10% bovine serum-containing DMEM medium were administered to the caudal vein of a unilateral ureteral ligation model rat. During the administration, phosphate-buffered saline (PBS) serving as a control was similarly administered to the caudal vein of the unilateral ureteral ligation model rat. Three days and six days after the administration of the mesenchymal stem cells, the unilateral ureteral ligation model rat was sacrificed and then a kidney thereof was collected.

A TGF-β1 expression level and an α-SMA expression level, each serving as an indicator of fibrosis, were checked in the collected kidney cell so that a fibrosis suppressing effect was evaluated.

An expression level of mRNA was checked in accordance with a well-known protocol of a real-time polymerase chain reaction (PCR) method. An expression level of a protein was checked in accordance with a well-known protocol of Western blotting. Fluorescent immunostaining was carried out with use of a commercially-available antibody in accordance with a well-known protocol.

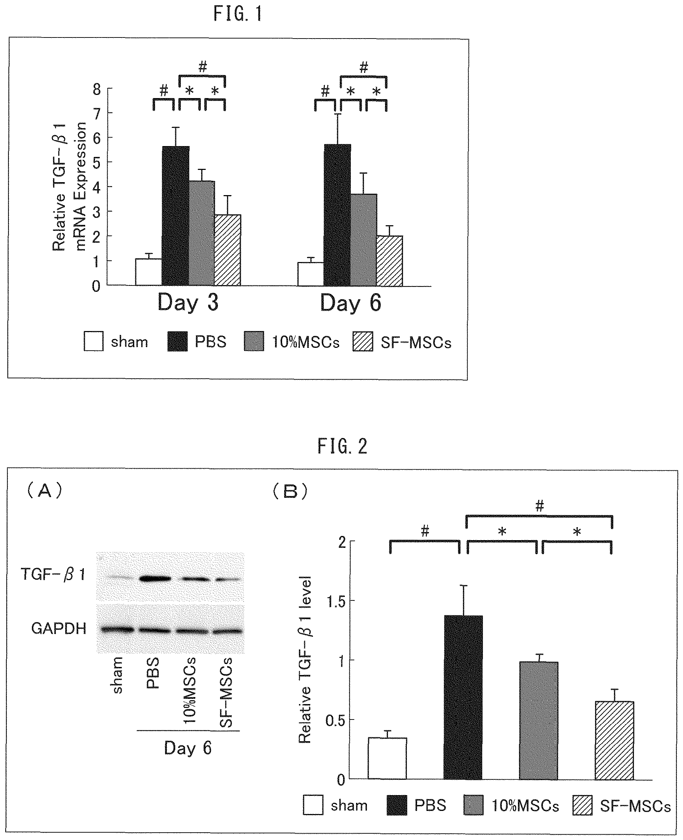

In each of Figs. 1 to 9, "sham" means "sham surgery", "PBS" means "a test result obtained in a case where PBS is administered", "10% MSCs" means "a test result obtained in a case where mesenchymal stem cells cultured in the 10% bovine serum-containing DMEM medium are administered", and "SF-MSCs" means "a test result obtained in a case where mesenchymal stem cells cultured in the serum-free medium STK2 are administered".

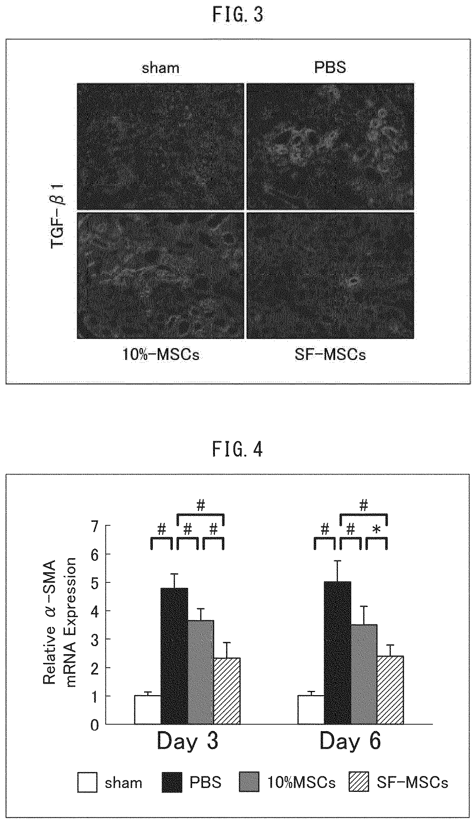

The test results of Figs. 1 to 3 were obtained under the following conditions: "n = 5 to 6", "*: P < 0.05", "#: P < 0.01", and "a magnification of 200 at which to carry out observation". The test results of Figs. 4 to 6 were obtained under the following conditions: "n = 5 to 6", "*: P < 0.05", "#: P < 0.01", and "a magnification of 200 at which to carry out observation". The test results of Figs. 7 to 9 were obtained under the following conditions: "n = 5 to 6", "*: P < 0.05", and "#: P < 0.01". The test results of Figs. 10 and 11 were obtained under the following conditions: "n = 6", "*: P < 0.05", and "#: P < 0.01".

Fig. 1 shows graphs showing TGF-β1 mRNA expression levels obtained 3 days (on Day 3) and 6 days (on Day 6) after administration of mesenchymal stem cells to a rat. As shown in the graphs of Day 3, a lower TGF-β 1 mRNA expression level was obtained in a case where the 10% MSCs were administered than in a case where the PBS serving as a control was administered. Furthermore, a significantly reduced TGF-β1 mRNA expression level was obtained in a case where the SF-MSCs were administered as compared with a case where the 10% MSCs were administered. A result similar to the above result was obtained on Day 6.

(A) of Fig. 2 shows a result of a test carried out with respect to a TGF-β by Western blotting. A lower TGF-β1 protein expression level was obtained in a case where the SF-MSCs were administered than in a case where the 10% MSCs were administered. (B) of Fig. 2 shows graphs each showing a TGF-β1 protein expression level. A lower TGF-β1 protein expression level was obtained in a case where the 10% MSCs were administered than in a case where the PBS serving as a control was administered. Furthermore, a significantly reduced TGF-β1 protein expression level was obtained in a case where the SF-MSCs were administered as compared with a case where the 10% MSCs were administered.

Fig. 3 shows an image showing a result of fluorescent immunostaining carried out in an Example of the present disclosure. TGF-β1 expression was strongly suppressed in a case where the SF-MSCs were administered.

Fig. 4 shows graphs each showing an amount of mRNA of α-SMA. As in the case of the result of TGF-β1 expression, α-SMA expression was strongly suppressed on Day 3 in a case where the SF-MSCs were administered. A result similar to the above result was obtained on Day 6.

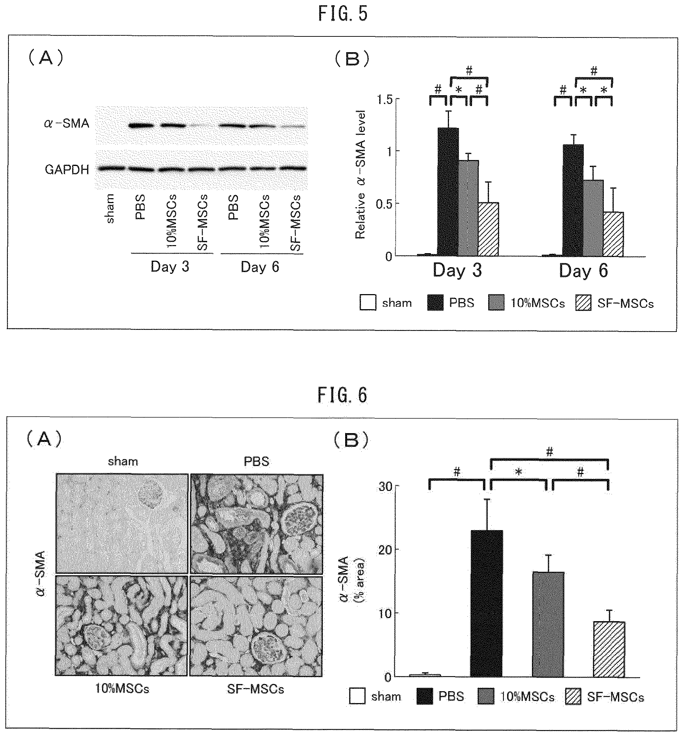

Fig. 5 shows a result of a test carried out with respect to a α-SMA by Western blotting. On both Day 3 and Day 6, a lowest α-SMA protein expression level was obtained in a case where the SF-MSCs were administered to the rat. (B) of Fig. 5 shows graphs each quantifying an amount of protein of α-SMA. On both Day 3 and Day 6, a significantly reduced amount of α-SMA was obtained in a case where the SF-MSCs were administered to the rat.

(A) of Fig. 6 shows an image showing a result of fluorescent immunostaining carried out in an Example of the present disclosure.

(B) of Fig. 6 shows a result of a count of the number of α-SMA positive cells per unit area of the image shown in (A) of Fig. 6. As is clear from (A) and (B) of Fig. 6, α-SMA positive cells were extremely small in number in a case where the SF-MSCs were administered to the rat.

The results of Figs. 1 to 6 showed that mesenchymal stem cells cultured in a serum-free medium are much more effective in suppression of fibrosis than mesenchymal stem cells cultured in a serum medium. That is, the mesenchymal stem cells cultured in a serum medium were less effective in suppression of fibrosis and insufficient to be used as a pharmaceutical agent. In contrast, the mesenchymal stem cells cultured in a serum-free medium were remarkably effective in suppression of fibrosis and sufficient to be used as a pharmaceutical agent.

### (Example 2)

Example 2 examined an effect, brought about by implantation of mesenchymal stem cells cultured in a serum-free medium, of suppressing infiltration of an inflammatory cell. A cell culture condition, a cell culture method, and the like of Example 2 are as described in Example 1. CD3, which is an indicator of T lymphocyte, and CD68, which is an indicator of a macrophage were used to evaluate infiltration of an inflammatory cell in a kidney of a rat whose ureter had been ligated.

(A) of Fig. 7 shows graphs showing CD3 and CD68 mRNA expression levels obtained 3 days (on Day 3) and 6 days (on Day 6) after administration of mesenchymal stem cells to the rat. As shown in the graphs of Day 3, a lower CD3 mRNA expression level was obtained in a case where 10% MSCs were administered than in a case where PBS serving as a control was administered. Furthermore, a significantly reduced CD3 mRNA expression level was obtained in a case where SF-MSCs were administered as compared with a case where the 10% MSCs were administered.

(B) of Fig. 7 shows graphs showing CD68 mRNA expression levels obtained 3 days (on Day 3) and 6 days (on Day 6) after administration of the mesenchymal stem cells to the rat. On both Day 3 and Day 6, a lower CD68 mRNA expression level was obtained in a case where the 10% MSCs were administered than in a case where PBS serving as a control was administered. Furthermore, a significantly reduced CD68 mRNA expression level was obtained in a case where the SF-MSCs were administered as compared with a case where the 10% MSCs were administered.

Fig. 8 shows an image showing a result of immunostaining carried out in an Example of the present disclosure. Black dots seen in the image indicate spots where CD3 and CD68 are expressed. Expression of both CD3 and CD68 was strongly suppressed in a case where the SF-MSCs were administered than as compared with a case where the PBS was administered or a case where the 10% MSCs were administered.

Fig. 9 shows results of a count of the number of CD3 positive cells and the number of CD68 positive cells per unit area of the image shown in Fig. 8. As in the case of the result of Fig. 8, CD3 positive cells and CD68 positive cells were extremely small in number in a case where the SF-MSCs were administered to the rat.

The results of Figs. 7 to 9 showed that mesenchymal stem cells cultured in a serum-free medium are much more effective in suppression of infiltration of inflammatory cells than mesenchymal stem cells cultured in a serum medium. That is, the mesenchymal stem cells cultured in a serum medium were less effective in suppression of infiltration of inflammatory cells and insufficient to be used as a pharmaceutical agent. In contrast, the mesenchymal stem cells cultured in a serum-free medium were remarkably effective in suppression of infiltration of inflammatory cells and sufficient to be used as a pharmaceutical agent.

### (Example 3)

Example 3 evaluated macrophage activity controllability exhibited by bone marrow-derived mesenchymal stem cells cultured in a serum-free medium.

Human monoblastoid cells (THP-1) were cultured and then the cells were stimulated by 160 nM PMA for 48 hours so as to be differentiated into Pro-inflammatory phenotype (M1) macrophages. Human bone marrow-derived mesenchymal stem cells (hMSCs: Bio Whittaker (Walkersville, MD)) were cultured in a 10% bovine serum-containing DMEM medium (10% MSCs) or a serum-free medium STK2 (SF-hMSCs) and used for an experiment in coculture with the THP-1 (Transwell experiment).

### <Coculture condition>

The 10% MSCs and the SF-hMSCs were cocultured with the THP-1 with use of a Transwell system. The THP-1 were collected 72 hours after the start of the coculture, and expression of CD163 and CD206, each serving as a marker of an Immune-regulatory phenotype (M2) macrophage, was determined by a FACS method.

Fig. 10 is a view showing induction of differentiation from an M1-type macrophage into an M2-type macrophage. Differentiation from an M1-type macrophage into an M2-type macrophage was further induced in a case where M1-type macrophages were cocultured with the SF-hMSCs than in a case M1-type macrophages were cocultured with the 10% hMSCs.

(A) of Fig. 11 shows graphs showing an increase in CD163 positive cell. (B) of Fig. 11 shows graphs showing an increase in CD206 positive cell.

The M1-type macrophages which were cocultured with the SF-MSCs were much larger in proportion of CD163 positive cells and in proportion of CD206 positive cells than the M1-type macrophages which were cocultured with the 10% hMSCs.

The results of Figs. 10 and 11 revealed that bone marrow-derived mesenchymal stem cells cultured in a serum-free medium cause a considerable change from an M1-type macrophage into an M2-type macrophage. That is, bone marrow-derived mesenchymal stem cells cultured in a serum medium were less effective in increase in inflammation-suppressing macrophage and insufficient to be used as a pharmaceutical agent. In contrast, the bone marrow-derived mesenchymal stem cells cultured in a serum-free medium were highly effective in increase in inflammation-suppressing macrophage and sufficient to be used as a pharmaceutical agent.

### (Example 4)

Example 4 evaluated macrophage activity controllability exhibited by adipose tissue-derived mesenchymal stem cells cultured in a serum-free medium.

Human monoblastoid cells (THP-1) were cultured and then the cells were stimulated by 160 nM PMA for 48 hours so as to be differentiated into Pro-inflammatory phenotype (M1) macrophages. Human adipose tissue-derived mesenchymal stem cells (hAMSCs: human adult subcutaneous adipose tissue-derived mesenchymal stem cells (Cat#: BBASCF))) were cultured in a 10% bovine serum-containing DMEM medium (10% hAMSCs) or a serum-free medium STK2 (SF-hAMSCs) and used for an experiment in coculture with THP-1 (Transwell experiment).

### <Coculture condition>

The 10% hAMSCs and the SF-hAMSCs were cocultured with the THP-1 with use of a Transwell system. The THP-1 were collected 72 hours after the start of the coculture, and expression of CD163 and CD206, each serving as a marker of an Immune-regulatory phenotype (M2) macrophage, was determined by a FACS method.

(A) of Fig. 12 shows graphs showing an increase in CD163 positive cell. (B) of Fig. 12 shows graphs showing an increase in CD206 positive cell.

M1-type macrophages which were cocultured with the SF-hAMSCs were much larger in proportion of CD163 positive cells and in proportion of CD206 positive cells than M1-type macrophages which were cocultured with the 10% hAMSCs.

A result of this revealed that not only the bone marrow-derived mesenchymal stem cells cultured in a serum-free medium but also adipose tissue-derived mesenchymal stem cells cultured in a serum-free medium cause a considerable change from an M1-type macrophage into an M2-type macrophage. That is, not only the bone marrow-derived mesenchymal stem cells cultured in a serum medium but also adipose tissue-derived mesenchymal stem cells cultured in a serum medium were less effective in suppression of inflammation and insufficient to be used as a pharmaceutical agent. In contrast, not only the bone marrow-derived mesenchymal stem cells cultured in a serum-free medium but also the adipose tissue-derived mesenchymal stem cells cultured in a serum-free medium were remarkably effective in suppression of inflammation and sufficient to be used as a pharmaceutical agent.

### (Example 5)

Example 5 evaluated an anti-inflammatory effect brought about by bone marrow-derived mesenchymal stem cells cultured in a serum-free medium. An anti-inflammatory effect brought about by a cell was checked with use of an HGF serving as an indicator of fibrosis, PGE2 serving as an indicator of kidney damage, IL-6 serving as an indicator of inflammation, and TSG-6 serving as an indicator of anti-inflammation.

A 10% FBS medium in which Human kidney-2 (HK-2) cells had been cultured, a 10% FBS-containing DMEM medium in which human MSCs (hMSCs) had been cultured, and STK2 in which hMSCs had been cultured were replaced with respective DMEM media each containing no FBS. Then, the Human kidney-2 (HK-2) cells, the human MSCs (hMSCs), and the hMSCs were cultured in the respective DMEM media for 48 hours. Thereafter, the DMEM media were collected. Subsequently, respective protein levels of an HGF and PGE2 which were contained in each of the DMEM media collected were determined by an Elisa method.

Furthermore, mRNA was collected from the hMSCs cultured in the 10% FBS-containing DMEM medium and from the hMSCs cultured in the STK2, and mRNA expression of IL-6 and TSG-6 was determined by RT-PCR.

In each of Figs. 13 to 16, "HK-2" means "a result of a test on HK-2 cultured in the 10% FBS medium", "10% MSCs" means "a result of a test on mesenchymal stem cells cultured in a 10% bovine serum-containing DMEM medium", and "SF-MSCs" means "a result of a test on mesenchymal stem cells cultured in the serum-free medium STK2".

Fig. 13 shows graphs each showing an HGF protein expression level. Fig. 14 shows graphs each showing a PGE2 protein expression level. The SF-MSCs were higher than the HK-2 but significantly lower than the 10% MSCs in HGF protein expression level and in PGE2 protein expression level.

Fig. 15 shows graphs each showing an IL-6 mRNA expression level. The 10% MSCs were significantly lower than the HK-2 in IL-6 mRNA expression level. In contrast, the SF-MSCs were even lower than the 10% MSCs in IL-6 mRNA expression level.

Fig. 16 shows graphs each showing a TSG-6 mRNA expression level. The SF-MSCs were approximately 4.5 times higher than the 10% MSCs in TSG-6 mRNA expression level. Furthermore, expression of TSG-6 mRNA was hardly observed in the HK-2.

Subsequently, hMSCs obtained by transfecting Negative Control siRNA (Santa Cruz Biotechnology) into the hMSCs cultured in the STK2 (seeded under a condition of 3 × 10⁵ cells/ 10 cm dish and cultured in the STK2 until the hMSCs are made 30% confluent) were prepared. Similarly, hMSCs obtained by transfecting TSG-6 siRNA (Santa Cruz Biotechnology) into the hMSCs cultured in the STK2 were prepared. Note here that 20 nM siRNA was transfected into each of the cells with use of Lipofectamine 2000 Transfection Reagent (Thermo Fisher Scientific). Twenty-four hours after the transfection, the medium was replaced with STK2 and the cells were cultured until they were made subconfluent. A transfection efficiency was examined by collecting mRNA from the above cells and determining the TSG-6 mRNA expression level by an RT-PCR method.

Fig. 17 shows graphs each showing a TSG-6 mRNA expression level. Note here that "NC siRNA/SF-hMSCs" shows "a test result obtained in a case where Negative Control siRNA is transfected into the hMSCs cultured in the STK2". In contrast, "TSG-6 siRNA/SF-MSCs" shows "a test result obtained in a case where TSG-6 siRNA is transfected into the hMSCs cultured in the STK2". Given that the TSG-6 mRNA expression level obtained in the TSG-6 siRNA/SF-MSCs is significantly reduced as compared with the TSG-6 mRNA expression level obtained in the NC siRNA/SF-hMSCs, it was confirmed that TSG-6 siRNA has a beneficial knockdown effect.

Next, human monoblastoid cells (THP-1) were cultured and then stimulated by 160 nM PMA for 48 hours. Similarly, 20 ng/mL of IFN-y was added to the THP-1, and the THP-1 to which the IFN-y had been added were stimulated for 24 hours so as to be differentiated into Pro-inflammatory phenotype (M1) macrophages. The NC siRNA/SF-hMSCs and the TSG-6 siRNA/SF-MSCs were cocultured with the THP-1 with use of a Transwell system. The THP-1 were collected 72 hours after the start of the coculture, expression of CD163, serving as a marker of an Immune-regulatory phenotype (M2) macrophage, in the THP-1 was determined by a FACS method.

Fig. 18 shows graphs showing an increase in CD163 positive cell. Note here that "NC siRNA/SF-hMSCs/TW" shows "a test result obtained in a case where Negative Control siRNA is transfected into the hMSCs cultured in the STK2". "TSG-6 siRNA/SF-MSCs/TW" shows "a test result obtained in a case where TSG-6 siRNA is transfected into the hMSCs cultured in the STK2". No significant difference can be observed between the TSG-6 siRNA/SF-MSCs/TW and the NC siRNA/SF-hMSCs/TW. That is, MSCs cultured with use of the STK2 cause a change from a Pro-inflammatory phenotype (M1) macrophage into an Immune-regulatory phenotype (M2) macrophage. Note, however, that it is suggested that the TSG-6, whose expression level is increased by the STK2, is not involved in the change.

As in the case of the method described earlier, a medium in which the hMSCs to which Negative Control siRNA had been administered and the hMSCs to which TSG-6 siRNA had been administered were cultured was replaced with a DMEM medium containing no FBS, and then the hMSCs to which Negative Control siRNA had been administered and the hMSCs to which TSG-6 siRNA had been administered were cultured in the DMEM medium containing no FBS for 48 hours (such a medium in which the hMSCs were cultured is hereinafter referred to as a "condition medium"). The hMSCs thus cultured were used for an experiment below.

HK-2 cells were seeded on a 6 well dish at 1×10⁵ cells/well, and it was confirmed that the HK-2 cells were made subconfluent 18 hours after the seeding. Then, the medium was replaced with the above condition medium. Twenty-four hours after the replacement, 10 ng/mL of a recombinant human TGF-β1 (R&D Systems, Minneapolis, MN, USA) was added. Twelve hours after the addition, mRNA was collected. Thereafter, mRNA expression of (i) MCP-1, which is inflammatory cytokine induced by a TGF-β1, and (ii) a TNF-α was studied with use of RT-PCR.

(A) of Fig. 19 shows graphs each showing an MCP-1 mRNA expression level. (B) of Fig. 19 shows graphs each showing a TNF-α mRNA expression level. The TGF-β1 increased the MCP-1 mRNA expression level and the TNF-α mRNA expression level in the HK-2 cells. Note here that "DMEM" shows "a test result obtained in a case where the HK-2 cells are cultured in the DMEM to which the TGF-β1 is added". Note also that "NC siRNA SF-hHMCs-CM" shows "a test result obtained in a case where the HK-2 cells are cultured in a culture solution containing the hMSCs to which Negative Control siRNA has been administered". Furthermore, "TSG-6 siRNA SF hMSCs-CM" shows "a test result obtained in a case where the HK-2 cells are cultured in a culture solution containing the hMSCs to which TSG-6 siRNA has been administered". In the NC siRNA SF-hHMCs-CM group, the MCP-1 mRNA expression level and the TNF-α mRNA expression level were significantly controlled. In contrast, in the TSG-6 siRNA SF hMSCs-CM group, an MCP-1 mRNA expression suppressing effect and a TNF-α mRNA expression suppressing effect were significantly reduced. That is, it was suggested that an effect of suppressing inflammatory cytokine induced by addition of the TGF-β1 is reduced in a case where TSG-6 which is highly expressed in the MSCs cultured in the STK2 is knocked down. A result obtained in Example 5 made it clear that mesenchymal stem cells cultured in a serum-free medium are remarkably highly effective in suppression of inflammation by expression of TSG-6 and sufficient enough to be used as a pharmaceutical agent.

### (Example 6)

MSCs collected from the tibia of a femur or the tibia of the bone marrow of a Sprague Dawley rat (6 weeks old to 7 weeks old) were isolated in a serum-free medium (STK1 or STK2) so as to be cultured and proliferated. Thus, MSCs cultured in a serum-free medium (hereinafter also referred to as "SF-MSCs") were obtained. Negative Control siRNA (20 nM) or TSG-6 siRNA (20 nM) was transfected into the MCSs. The cells which had been subjected to the transfection were administered to the caudal vein of a unilateral tubular ligation model rat which had been prepared 4 days before the administration. Example 6 prepared 5 groups of rats to which SF-MSCs into which the Negative Control siRNA had been transfected were administered, 5 groups of rats to which SF-MSCs into which the TSG-6 siRNA had been transfected were administered, 5 groups of rats to which only PBS was administered, and 5 groups of control rats whose tubules were not ligated. The rats of each of the groups were sacrificed 10 days after unilateral tubular ligation surgery, and a kidney was taken out, so that an anti-inflammatory effect of TSG-6 was confirmed.

Fig. 20 shows graphs each showing a TSG-6 mRNA expression level. Not only in the case of the human MSCs but also in the case of rat MSCs, SF-MSCs significantly increased in TSG-6 mRNA level as compared with MSCs cultured in a serum-containing medium (10% MSCs). Given that the TSG-6 mRNA level obtained in the SF-MSCs to which the TSG-6 siRNA had been administered was significantly reduced as compared with the TSG-6 mRNA level obtained in the SF-MSCs to which the Negative Control siRNA had been administered, it was confirmed that the TSG-6 siRNA has a knockdown effect.

(A) of Fig. 21 shows an image showing a result of detection of a TGF-β1 or α-SMA by Western blotting. (B) of Fig. 21 shows graphs showing TGF-β1 expression levels and α-SMA expression levels. Test results of (B) of Fig. 21 were obtained under the following conditions: "n = 5", "*: P < 0.05", and "#: P < 0.01". A PBS group had the highest TGF-β1 protein expression level and the highest α-SMA protein expression level. Furthermore, a group to which NC siRNA/SF-MSCs had been administered had a significantly reduced TGF-β1 protein expression level and a significantly reduced α-SMA protein expression level. In contrast, a group in which the TSG-6 had been knocked down and to which TSG-6 siRNA/SF-MSCs had been administered had a higher TGF-β1 protein expression level and a higher α-SMA protein expression level than the group to which the NC siRNA/SF-MSCs had been administered. That is, it was found that an effect of suppressing expression of a TGF-β1 protein and an α-SMA protein is significantly reduced in a case where the TSG-6 is knocked down.

(A) of Fig. 22 shows an image showing a result of immunostaining of CD68. (B) of Fig. 22 shows graphs each showing the number of cells in which CD68 is expressed. The PBS group had the largest number of CD68 positive cells of all the groups. Furthermore, a significant reduction in number of CD68 positive cells was observed in the NC siRNA/SF-MSCs group. The TSG-6 siRNA/SF-MSCs group was observed to significantly increase in CD68 positive cell as compared with the NC siRNA/SF-MSCs group.

It was made clear that TSG-6 capable of relieving inflammation early is involved in an anti-inflammatory action and an antifibrotic action of mesenchymal stem cells cultured in a serum-free medium.

### (Example 7)

As fibrosis of a liver advances, extracellular matrices containing collagen as a main component are excessively deposited in a liver tissue. This finally leads to a fibrotic liver (e.g., cirrhosis, viral hepatitis, alcoholic hepatitis, or non-alcoholic steatohepatitis). Example 7 carried out a study on an effect, brought about by implantation of mesenchymal stem cells cultured in a serum-free medium, of suppressing fibrosis of a liver (e.g., cirrhosis, viral hepatitis, alcoholic hepatitis, or non-alcoholic steatohepatitis).

### <Method: collection and culture of cell>

In the present test, by a method similar to the method described in Example 1, mesenchymal stem cells were collected from a femur or the tibia of Sprague Dawley rats (7 weeks old, male), and the mesenchymal stem cells were cultured and subcultured.

### <Method: preparation of model of disease>

As a common model of fibrosis of the liver, model rats treated with carbon tetrachloride were prepared.

In the present test, Sprague Dawley rats (5 weeks old, male) were acclimatized for 1 week, and then from when each of the rats became 6 weeks old, a disease inducing agent (disease inducing substance: carbon tetrachloride (40 w/w%), solvent: corn oil) was intraperitoneally administered to the rat over 5 weeks 10 times in total.

In each of the weeks, the disease inducing agent was administered to the rat at regular intervals 2 times a week (for example, 2 times on Day 0 and Day 2 in the first week, and 2 times on Day 28 and Day 31 in the fifth week).

The disease inducing agent was administered at each of the times in an amount (2 ml/kg) standardized based on a body weight of each individual. Note that a day serving as a starting point of reckoning the number of days (i.e., Day 0) was regarded as a day of initial administration of the disease inducing agent. A group which was subjected to such disease induction as described earlier is hereinafter referred to as a "disease induction group". Furthermore, a "healthy group" serving as a negative control to such a model for fibrosis of a liver as described earlier was separately provided. The "healthy group" was prepared by administering only a solvent, containing no disease inducing substance, to the Sprague Dawley rat, in a time course and an amount which are similar to those described earlier.

### <Method: administration of test substance/ placebo>

The "disease induction group", which consisted of disease model animals, was subjected to partial blood collection on the day (Day 32) following the day (Day 31) on which the final administration of the disease inducing agent had been carried out.

Rats were selected so that the value of aspartate aminotransferase (AST) and the value of body weight could be considered to be uniform also in the subgrouping described later, and the selected rats were assigned to the "disease induction group".

Animals belonging to the "disease induction group" were divided into three groups, which are an "STK group" (n = 5), a "serum-containing group" (n = 8), and a "placebo group" (n = 11). The "STK group" and the "serum-containing group" are hereinafter collectively referred to as a "cell administration group".

On Day 33, mesenchymal stem cells cultured with use of STK1 and STK2 by the method described in Example 1 were injected into the caudal vein of an animal belonging to the "STK group", whereas mesenchymal stem cells cultured with use of a 10% fetal bovine serum-containing DMEM medium by the method described in Example 1 were injected into the caudal vein of an animal belonging to the "serum-containing group".

To the "cell administration group", a suspension obtained by suspending cells in phosphate-buffered saline (PBS) at a concentration of 2×10⁶ cells/ml was administered at 500 pL/rat (consequently, 1×10⁶ cells were administered to each rat).

To an animal belonging to the "placebo group", placebos (only PBS) were similarly administered at 500 pL/rat. The administration to each of the animals was carried out by an ordinary method within several seconds while a rat was retained. Note that neither cells nor placebos were administered to the "healthy group".

### <Method: sacrifice and material collection>

On Day 47 after a two-week observation period following administration of a test substance/placebo, all the groups were subjected to sacrifice (bloodletting euthanasia) and material collection under isoflurane anesthesia. During the material collection, a medial lobe and a left lateral lobe of a liver to be subjected to a histological evaluation were collected, and, in addition, a blood sample to be subjected to a biochemical test was collected (whole blood was collected). The collected medial lobe and the collected left lateral lobe of the liver were fixed with use of formalin and then were embedded in a paraffin block. Thereafter, the paraffin block was cut thin so that a Sirius red stained specimen was prepared.

### <Result: behavior observation>

In the "disease induction group", over a period of administration of the disease inducing agent from Day 0 to Day 31, there was obtained an observational finding assumable in association with administration of the present disease inducing agent, such as an observational finding that all individuals crouched every time immediately after administration of the disease inducing agent. Such an observational finding was not obtained in the "healthy group". In the "cell administration group", no abnormal finding (e.g., rejection) that should be considered in cell therapy was obtained during an evaluation period having lasted over two weeks after cell administration.

### <Result: change in body weight>

A change in body weight of each group from the day of initial administration (Day 0) to the day on which the sacrifice was carried out (Day 47) is as shown in Fig. 23. Note that in each of Fig. 23 and Figs. 24 to 26 (described later), (a) shows a result of a test carried out with respect to the "healthy group", (b) shows a result of a test carried out with respect to the "placebo group", (c) shows a result of a test carried out with respect to the "serum-containing group", and (d) shows a result of a test carried out with respect to the "STK group".

On the day of initial administration of the disease inducing agent (Day 0), the groups including the healthy group were identical in average body weight. On Day 33, subgrouping is carried out so that the groups different from the "healthy group" are identical in body weight. In the two-week observation period following cell administration, respective body weights of the groups are in the following relationship: "STK group" = "healthy group" > "serum-containing group" = "placebo group".

Model rats which are treated with carbon tetrachloride commonly tend to further lose body weight as compared with rats which are ordinarily bred. The fact that the "STK group" and the "healthy group" are substantially identical in body weight shows that a strong recovery of a systemic health condition is shown in the "cell administration group" (in particular, the "STK group").

### <Result: biochemical test>

From a blood sample obtained on Day 47, a serum was separated. In the following description, "AST" refers to aspartate aminotransferase, "LDH" refers to lactase dehydrogenase, and "y-GTP" refers to γ-glutamyl transpeptidase.

"LDH" and "y-GTP" of the "cell administration group" (in particular, the "STK group") improved to respective values equal to or less than those of "LDH" and "y-GTP" of the "healthy group". In the "healthy group", respective enzyme activity values of "LDH" and "y-GTP" each showed a tendency to be greater than a normal value by administration of a solvent (i.e., corn oil).

AST: Respective values of "AST" of the "STK group" (n = 5), the "serum-containing group" (n = 8), and the "placebo group" (n = 11) were significantly reduced from the values obtained at the time of the subgrouping (on Day 32) (P < 0.01). All the groups belonging to the "disease induction group" and the "healthy group" were observed to have no difference therebetween in value of "AST" contained in the blood sample obtained on Day 47. In all the groups belonging to the "disease induction group", the value of "AST" was reduced so as to fall within the range of a normal value.

LDH: A value of "LDH" obtained on Day 47 was measured. The "STK group" had an average of "LDH" of 946.2 U/L, the "serum-containing group" had an average of "LDH" of 1086.4 U/L, the "placebo group" had an average of "LDH" of 1027.9 U/L, and the "healthy group" had an average of "LDH" of 852.7 U/L (see Fig. 24). The value of "LDH" was further reduced in the "STK group" than in the "serum-containing group" and the "placebo group".

γ-GTP: A value of "y-GTP" obtained on Day 47 was measured. The "STK group" had an average of "y-GTP" of 0.9 U/L, the "serum-containing group" had an average of "y-GTP" of 1.09 U/L, the "placebo group" had an average of "y-GTP" of 1.26 U/L, and the "healthy group" had an average of "y-GTP" of 1. 16 U/L. The value of "y-GTP" was further reduced in the "STK group" than in the "serum-containing group" and the "placebo group" (see Fig. 25).

### <Result: histological evaluation>

Sirius red stained specimens of each lobe (right lobe and left lobe) obtained from each rat were prepared. The fibrosis areas were calculated from microscope images, captured at an object lens magnification of 10, of 6 fields around the central vein (2 lobes × 3 fields) by using an image analyzing device.

In a Sirius red stained image, a part on which fibrotic collagen has been deposited is stained red (in monochrome images of (a) to (d) of Fig. 26, a black linear part corresponds to a part stained red). A fibrosis ratio of each of the visual fields ((area of fibrosis / area of visual field) × 100) was found with respect to each of these images.

(a) of Fig. 26 shows a Sirius red stained image of the "healthy group", (b) of Fig. 26 shows a Sirius red stained image of the "placebo group", (c) of Fig. 26 shows a Sirius red stained image of the "serum-containing group", and (d) of Fig. 26 shows a Sirius red stained image of the "STK group". In the respective Sirius red stained images, the "healthy group" had a fibrosis ratio of 1.1%, the "placebo group" had a fibrosis ratio of 4.4%, the "STK group" had a fibrosis ratio of 1.1%, the "serum-containing group" had a fibrosis ratio of 2.4%, and thus the "STK" group had a lower fibrosis ratio than the "placebo group" and the "serum-containing group" (see Fig. 26).

### <Conclusion of Example 7>

In Example 7, in the "STK group", no abnormal finding (e.g., rejection) that should be considered in cell therapy was obtained after cell administration. Thus, a repair agent of an aspect of the present disclosure is considered to be highly safe.

In general, a degree of inflammation was low in Example 7. However, the values of "LDH" and "y-GTP" were reduced in the "STK group". Furthermore, respective body weights of the groups are in the following relationship: "STK group" = "healthy group" > "serum-containing group" = "placebo group". This shows that the body weight of the "STK group" recovered to a body weight close to the body weight of the "healthy group". The above facts show that in the "STK group", inflammation of a liver was suppressed and a systemic health condition was recovered.

### Industrial Applicability

The present disclosure can be suitably used for regenerative medicine whose object is to achieve recovery from tissue damage.

## Claims

1. A repair agent for use in repairing damaged tissue, comprising mesenchymal stem cells cultured in a serum-free medium, wherein the damaged tissue is tissue damage that accompanies chronic renal failure, a chronic kidney disease, cirrhosis, viral hepatitis, alcoholic hepatitis, or non-alcoholic steatohepatitis.

2. The repair agent for use in repairing damaged tissue as set forth in claim 1, wherein the repair agent suppresses fibrosis of a living tissue.

3. The repair agent for use in repairing damaged tissue as set forth in claim 1, wherein the repair agent suppresses infiltration of an inflammatory cell.

4. The repair agent for use in repairing damaged tissue as set forth in claim 1, wherein the repair agent controls activity of a macrophage.

5. The repair agent for use in repairing damaged tissue as set forth in any one of claims 1 through 4, wherein the serum-free medium contains an FGF, a PDGF, an EGF, at least one phospholipid, and at least one fatty acid.

6. The repair agent for use in repairing damaged tissue as set forth in any one of claims 1 through 5, wherein the mesenchymal stem cells are mesenchymal stem cells which have been selected by screening and are non-tumorigenic.

7. The repair agent for use in repairing damaged tissue as set forth in any one of claims 1 through 6, wherein the mesenchymal stem cells have been subcultured at least once.

8. The repair agent for use in repairing damaged tissue as set forth in claim 7, wherein the mesenchymal stem cells which are being subcultured are detached with use of a cell detaching agent containing neither a mammal-derived component nor a microorganism-derived component.

9. The repair agent for use in repairing damaged tissue as set forth in any one of claims 1 through 8, wherein the mesenchymal stem cells are cultured with use of a culture vessel suitable for culture of the mesenchymal stem cells.

10. A repair agent for use in repairing damaged tissue, comprising TSG-6,
the damaged tissue being tissue damage that accompanies chronic renal failure or a chronic kidney disease.

11. The repair agent for use in repairing damaged tissue as set forth in claim 10, wherein the repair agent suppresses fibrosis of a living tissue.

12. The repair agent for use in repairing damaged tissue as set forth in claim 10, wherein the repair agent suppresses infiltration of an inflammatory cell.

## Patentansprüche

1. Reparaturmittel zur Verwendung bei der Reparatur von geschädigtem Gewebe, umfassend mesenchymale Stammzellen, kultiviert in einem serumfreien Medium, wobei das geschädigte Gewebe ein Gewebeschaden ist, der mit chronischem Nierenversagen, einer chronischen Nierenerkrankung, Zirrhose, viraler Hepatitis, alkoholischer Hepatitis oder nichtalkoholischer Steatohepatitis einhergeht.

2. Reparaturmittel zur Verwendung bei der Reparatur von geschädigtem Gewebe gemäß Anspruch 1, wobei das Reparaturmittel Fibrose eines lebenden Gewebes unterdrückt.

3. Reparaturmittel zur Verwendung bei der Reparatur von geschädigtem Gewebe gemäß Anspruch 1, wobei das Reparaturmittel Infiltration einer Entzündungszelle unterdrückt.

4. Reparaturmittel zur Verwendung bei der Reparatur von geschädigtem Gewebe gemäß Anspruch 1, wobei das Reparaturmittel Aktivität eines Makrophagen kontrolliert.

5. Reparaturmittel zur Verwendung bei der Reparatur von geschädigtem Gewebe nach einem beliebigen der Ansprüche 1 bis 4, wobei das serumfreie Medium einen FGF, einen PDGF, einen EGF, mindestens ein Phospholipid und mindestens eine Fettsäure enthält.

6. Reparaturmittel zur Verwendung bei der Reparatur von geschädigtem Gewebe gemäß einem beliebigen der Ansprüche 1 bis 5, wobei die mesenchymalen Stammzellen mesenchymale Stammzellen sind, die durch Screening ausgewählt wurden und nicht tumorigen sind.

7. Reparaturmittel zur Verwendung bei der Reparatur von geschädigtem Gewebe nach einem beliebigen der Ansprüche 1 bis 6, wobei die mesenchymalen Stammzellen mindestens einmal subkultiviert worden sind.

8. Reparaturmittel zur Verwendung bei der Reparatur von geschädigtem Gewebe gemäß Anspruch 7, wobei die mesenchymalen Stammzellen, die subkultiviert sind, unter Verwendung eines Zellablösungsmittels abgelöst werden, das weder einen von einem Säuger stammenden Bestandteil noch einen von einem Mikroorganismus stammenden Bestandteil enthält.

9. Reparaturmittel zur Verwendung bei der Reparatur von geschädigtem Gewebe nach einem beliebigen der Ansprüche 1 bis 8, wobei die mesenchymalen Stammzellen unter Verwendung eines Kulturgefäßes kultiviert werden, das für Kultur der mesenchymalen Stammzellen geeignet ist.

10. Reparaturmittel zur Verwendung bei der Reparatur von geschädigtem Gewebe, umfassend TSG-6, wobei das geschädigte Gewebe ein Gewebeschaden ist, der mit chronischem Nierenversagen oder einer chronischen Nierenerkrankung einhergeht.

11. Reparaturmittel zur Verwendung bei der Reparatur von geschädigtem Gewebe gemäß Anspruch 10, wobei das Reparaturmittel Fibrose in einem lebenden Gewebe unterdrückt.

12. Reparaturmittel zur Verwendung bei der Reparatur von geschädigtem Gewebe gemäß Anspruch 10, wobei das Reparaturmittel Infiltration einer Entzündungszelle unterdrückt.

## Revendications

1. Agent de réparation pour utilisation dans la réparation d'un tissu endommagé, comprenant des cellules souches mésenchymateuses cultivées dans un milieu sans sérum, dans lequel le tissu endommagé est une lésion tissulaire qui accompagne une insuffisance rénale chronique, une maladie des reins chronique, une cirrhose, une hépatite virale, une hépatite alcoolique ou une stéatohépatite non alcoolique.

2. Agent de réparation pour utilisation dans la réparation d'un tissu endommagé selon la revendication 1, dans lequel l'agent de réparation supprime une fibrose d'un tissu vivant.

3. Agent de réparation pour utilisation dans la réparation d'un tissu endommagé selon la revendication 1, dans lequel l'agent de réparation supprime une infiltration d'une cellule inflammatoire.

4. Agent de réparation pour utilisation dans la réparation d'un tissu endommagé selon la revendication 1, dans lequel l'agent de réparation régule l'activité d'un macrophage.

5. Agent de réparation pour utilisation dans la réparation d'un tissu endommagé selon l'une quelconque des revendications 1 à 4, dans lequel le milieu sans sérum contient un FGF, un PDGF, un EGF, au moins un phospholipide, et au moins un acide gras.

6. Agent de réparation pour utilisation dans la réparation d'un tissu endommagé selon l'une quelconque des revendications 1 à 5, dans lequel les cellules souches mésenchymateuses sont des cellules souches mésenchymateuses qui ont été sélectionnées par criblage et sont non tumorigènes.

7. Agent de réparation pour utilisation dans la réparation d'un tissu endommagé selon l'une quelconque des revendications 1 à 6, dans lequel les cellules souches mésenchymateuses ont été sous-cultivées au moins une fois.

8. Agent de réparation pour utilisation dans la réparation d'un tissu endommagé selon la revendication 7, dans lequel les cellules souches mésenchymateuses qui sont en cours de sous-culture sont détachées à l'aide d'un agent de détachement de cellules ne contenant ni de composant dérivé d'un mammifère ni de composant dérivé d'un micro-organisme.

9. Agent de réparation pour utilisation dans la réparation d'un tissu endommagé selon l'une quelconque des revendications 1 à 8, dans lequel les cellules souches mésenchymateuses sont cultivées à l'aide d'un récipient de culture approprié pour la culture des cellules souches mésenchymateuses.

10. Agent de réparation pour utilisation dans la réparation d'un tissu endommagé, comprenant du TSG-6,
le tissu endommagé étant une lésion tissulaire qui accompagne une insuffisance rénale chronique ou une maladie des reins chronique.

11. Agent de réparation pour utilisation dans la réparation d'un tissu endommagé selon la revendication 10, dans lequel l'agent de réparation supprime une fibrose d'un tissu vivant.

12. Agent de réparation pour utilisation dans la réparation d'un tissu endommagé selon la revendication 10, dans lequel l'agent de réparation supprime une infiltration d'une cellule inflammatoire.
